# EUROPEAN PATENT SPECIFICATION

(11) **EP 2 953 951 B1**
(45) Date of publication and mention of the grant of the patent: **03.05.2017**
(21) Application number: 14710788.2
(22) Date of filing: 05.02.2014
(51) Int. Cl.: C07D 473/16, A61K 31/52, A61P 35/00

(54) **N2-SUBSTITUTED-N6-(6-ARYL-PYRIDINE-3-YLMETHYL)-9-CYCLOPENTYL-9H-PURINE-2,6-DIAMINE DERIVATIVES AS TUMOR SUPPRESSOR P53 ACTIVATORS FOR INHIBITING ANGIOGENESIS AND FOR TREATING CANCER**
N2-SUBSTITUIERTE-N6-(6-ARYL-PYRIDIN-3-YLMETHYL)-9-CYCLOPENTYL-9H-PURIN-2,6-DIAMIN-DERIVATE ALS TUMOR SUPPRESSOR P53 AKTIVATOREN ZUR ANGIOGENESE-HEMMUNG UND ZUR BEHANDLUNG VON KREBS
DÉRIVÉS 2,6-DIAMINES DE 9H-PURINE N2-SUBSTITUTÉS-N6-(6-ARYL-PYRIDINE-3-YLMÉTHYL)-9-CYCLOPENTYL EN TANT QUE ACTIVATEURS DU SUPRESSEUR DE TUMEUR P53 POUR INHIBER L'ANGIOGÉNÈSE ET POUR TRAITER DU CANCER

(30) Priority: 08.02.2013 CZ 20130088
(43) Date of publication of application: 16.12.2015
(73) Proprietor: Univerzita Palackeho v Olomouci, 77200 Olomouc (CZ); Biopatterns s.r.o., 77900 Olomouc, Holice (CZ)
(72) Inventor: GUCKY, Tomas, 78501 Mladejovice (CZ); JORDA, Radek, 77900 Olomouc (CZ); ZATLOUKAL, Marek, 78701 Sumperk (CZ); KRYSTOF, Vladimir, 77900 Olomouc (CZ); RAROVA, Lucie, 74258 Pribor (CZ); REZNICKOVA, Eva, 77900 Olomouc (CZ); MIKULITS, Wolfgang, A-1140 Wien (AT); STRNAD, Miroslav, 77900 Olomouc (CZ)
(74) Representative: Hartvichova, Katerina
(86) International application number: PCT/CZ2014/000014
(87) International publication number: WO 2014/121764

(56) References cited:
- WO-A1-00/44750
- WO-A1-00/55161
- WO-A2-03/022216
- WO-A2-03/022219
- WO-A2-03/022805
- SCHOW STEVEN R ET AL: "Synthesis and activity of 2,6,9-trisubstituted purines", BIOORGANIC & MEDICINAL CHEMISTRY LETTERS, PERGAMON, AMSTERDAM, NL, vol. 7, no. 21, 1 January 1997 (1997-01-01), pages 2697-2702, XP002481170, ISSN: 0960-894X, DOI: 10.1016/S0960-894X(97)10076-2

## Description

### Field of Art

The present invention relates to novel 2-substituted-6-biarylmethylamino-9-cyclopentyl-9H-purine derivatives, to their activity as specific inhibitors of growth and angiogenesis of hepatocellular carcinomas, and to their use as medicaments.

### Background Art

Hepatocellular carcinoma (HCC) belongs to the most common malignancies worldwide (El-Serag & Rudolph, 2007, Gastroenterology, 132(7):2557-76). Among the most common factors for HCC are included infections with hepatitis viruses, chronic excessive alcohol consumption, environmental toxins, hemochromatosis, α1-antitrypsin deficiency or nonalcoholic fatty liver diseases (Farazi & DePinho, 2006, Nat Rev Cancer, 6(9):674-87). Except of curative treatment of HCC by surgical resection or liver transplantation, targeted molecular-based therapy was recently established as a promising therapeutic option. Several chemotherapeutic agents are currently studied in a single agent therapy or in targeted therapy in tandem or combination with other conventional agents (Chua & Choo, 2011, Int J Hepatol 348297. Epub 2011 Jul 12; Tanaka & Arii, 2011, J Gastroenterol, 46(3):289-96). Unfortunately most agents have shown a limited activity in HCC probably due to a relatively high chemoresistance of this type of tumor.

Inhibition of angiogenesis has been considered as a rational treatment strategy due to a typical hypervascularization of HCC nodules in tumors (Welker & Trojan, 2011, World J Gastroenterol, 14;17(26):3075-81) but recently other molecular targets, including cancer stem cells, have been established as next potential strategies of HCC treatment (Tanaka & Arii, 2011, J Gastroenterol 46(3):289-96). Sorafenib (Nexavar) was approved as a first drug for treatment of advanced stage HCC, targeting a broad spectrum of kinases including VEGFR, PDGFR and Raf that are frequently hyperactivated in HCC. Unfortunately, exact indication of sorafenib is still broad and unclear (Kim et al., 2011, Oncology, 25(3):283-91, 295) so therefore novel therapeutic strategies for efficient treatment of HCC are critically needed.

Recently, therapy by transarterial chemoembolisation (TACE) has been introduced for treatment of unresectable HCC (Llovet & Bruix, 2008, J Hepatol, 48 Suppl 1:S20-37) that effectively leads to selective distribution and a higher retention time of frequently used chemotherapeutics such as doxorubicin, cisplatin and epirubicin in the tumor (Llovet & Bruix, 2003 Hepatology, 37(2):429-42; Marelli et al., 2006, Cancer Treat Rev, 32(8):594-606).

Also new findings in pathology of malignant hepatocytes reflecting the specifity of HCC progression have been established (Zijl et al., 2009, Future Oncol, 5(8):1169-79). The recently established human model of epithelial to mesenchymal transition (EMT) emphasises the importance of this process especially in cancer extension, metastatic colonization, and useful evaluation of drug efficacy during HCC progression (Zijl et al., 2011, Mol Cancer Ther,10(5):850-60).

Substitution of the purine ring in positions 2, 6, and 9 by a wide range of substituents was carried out and these compounds were tested, e.g., substitution by benzyl and phenyl based substituents in position N⁶ (WO 03/040144, WO2010CZ00067, WO2010CZ00004), substitution by ribose in position 9 (WO 2004/058791), substitution by less sterically demanding substituents in position 2 (WO 2009/003428), substitution by 2-substituted benzyl substituents in position N⁶ (WO 2009/043320). US696970B2 relates to 2,6,9-trisubstituted biaryl purine derivatives, bearing mainly short alkyl in position 9 (methyl, ethyl, isopropyl), and to their use as CDK1/2 inhibitors and antiproliferative compounds. WO 03/022216A2 and WO 00/55161A1 also relate to 2,6,9-disubstituted biaryl adenine derivatives, bearing isopropyl moiety in position 9, and to their use in several hyperproliferative diseases. These substitutions, however, did not result in useful drugs against hepatocellular carcinoma.

Documents WO03022805, WO03022219, WO03022216 and WO0055161 show 2-substituted-6-biarylalkylamino-9-alkyl purines as kinase inhibitors. These compounds have a lower inhibitory activity than the compounds of the present invention. Documents WO0044750 and Schow et al. Bioorg. Med. Chem. Lett., Vol. 7, No. 21, pp. 2697-2702 (1997) show further 2,6,9-trisubstituted purine derivatives as kinase inhibitors which are substitution derivatives of a known inhibitor benzylaminopurine.

The present invention therefore provides a series of novel 2-substituted-6-biarylmethylamino-9-cyclopentyl-9*H*-purine derivatives that are useful for inhibition of growth as well as angiogenesis of hepatocellular carcinoma. This group of new purine derivatives is characterised by an unusual combination of cytotoxic, antiangiogenic, antiinflammatory and proapoptotic activity thus bringing not only strong anticancer properties to the compounds but also heretofore uknown type of activities (antiangiogenic, proapoptiotic, anti-inflammatory) useful for treatment of hepatocarcinoma, in particular targeted to metastatic hepatocellular carcinoma. It is the aim of this invention to provide a new generation of unique and effective anticancer compounds having improved selectivity and efficiency index.

### Disclosure of the Invention

Object of the present invention are substituted 2-substituted-6-biarylmethylamino-9-cyclopentyl-9*H*-purines of the general formula **I** 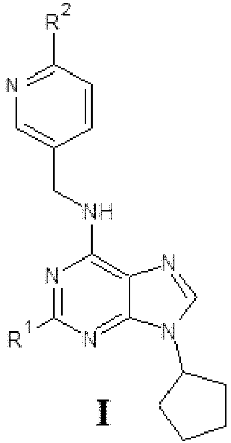 wherein
R1 is selected from the group consisting of
   (4- aminocyclohexyl) amino,
   (2-aminocyclohexyl) amino,
   (3-aminocyclohexyl) amino,
   (4-hydroxycyclohexyl)amino,
   [(2*R*)-1-hydroxybutan-2-yl] amino,
   (2-hydroxy-2-methylpropyl)amino,
   (3-hydroxy-3-methylbutyl)amino,
   [(3*S*)-2-hydroxy-2,4-dimethylpent-3-yl]amino,
   [(1*S*)-1-(dimethylamino)-2-hydroxyethyl] amino,
   [(3*R*)-2-hydroxypent-3-yl]amino,
   (3-hydroxypropyl)amino,
   (2-aminoethyl)amino,
   (3-aminopropyl)amino,
   (2-aminopropyl) amino,
   and
R2 is selected from the group consisting of
   substituted phenyl, wherein the substituents are in any position and are independently selected from the group consisting of OH, OCH₃, NH₂, Cl, Br, F, I, COOH, NO₂,
   2-pyridyl,
   3-pyridyl,
   4-pyridyl,
   2-furanyl
   3-furanyl,
   thien-2-yl,
   thien-3-yl,
   pyrazol-1-yl,
   pyrazol-3-yl,
   pyrazol-4-yl,
   pyrrol-1-yl,
   and the pharmaceutically acceptable salts thereof, in particular salts with alkali metals, ammonium or amines, or addition salts with acids.

When chiral centers are present in the molecule, the present invention encompasses optically active isomers, their mixtures and racemates.

Another object of this invention are 2-substituted-6-biarylmethylamino-9-cyclopentyl-9*H*-purines of the general formula I for use as medicaments.

A further object of this invention are 2-substituted-6-biarylmethylamino-9-cyclopentyl-9*H*-purines of the general formula I for use in inhibiting cell proliferation and/or inducing apoptosis.

Yet another object of this invention are 2-substituted-6-biarylmethylamino-9-cyclopentyl-9*H*-purines of the general formula I for use in inhibiting angiogenesis.

A further object of this invention are 2-substituted-6-biarylmethylamino-9-cyclopentyl-9*H*-purines of the general formula I for use as antiinflammatory compounds.

Yet further object of this invention are 2-substituted-6-biarylmethylamino-9-cyclopentyl-9*H*-purines of the general formula **I** for use in the treatment of cancer disorders, preferably selected from the group comprising hepatocellular carcinoma and metastatic hepatocellular carcinoma. In particular, these compounds combine antiproliferative, antiangiogenic, antiinflammatory and proapoptotic activities.

Another object of this invention are 2-substituted-6-biarylmethylamino-9-cyclopentyl-9*H*-purines of the general formula **I** for use in the manufacture of medicaments for the treatment of cancer disorders, such as tumors.

The compounds of the present invention are inhibitors of cyclin-dependent kinases (CDKs) selected from the group comprising CDK 5, 7 and 9 and erk1 or combinations thereof. They also activate the tumor supressor p53.

The invention also includes a pharmaceutical composition comprising at least one 2-substituted-6-biarylmethylamino-9-cyclopentyl-9*H*-purines of the general formula **I**, and a pharmaceutically acceptable carrier, and optionally another anticancer agent selected from the group compising cis-platin, doxorubicin or sorafenib.

In a preferred embodiment, the derivatives of formula **I** are selected from the group consisting of:
*N*²-(4-amino-cyclohexyl)-*N⁶*-[2,2']bipyridinyl-5-ylmethyl-9-cyclopentyl-9*H*-purine-2,6-diamine, *N*²-(4-amino-cyclohexyl)-9-cyclopentyl-*N*⁶-(6-thiophen-2-yl-pyridin-3-ylmethyl)-9*H*-purine-2,6-diamine, *N*²-(4-amino-cyclohexyl)-9-cyclopentyl-*N*⁶-(6-thiophen-3-yl-pyridin-3-ylmethyl)-9*H*-purine-2,6-diamine, *N*²-(4-amino-cyclohexyl)-9-cyclopentyl-*N*⁶-(6-furan-2-yl-pyridin-3-ylmethyl)-9*H*-purine-2,6-diamine, *N*²-(4-amino-cyclohexyl)-9-cyclopentyl-*N*⁶-(6-furan-3-yl-pyridin-3-ylmethyl)-9*H*-purine-2,6-diamine, *N*²-(4-amino-cyclohexyl)-9-cyclopentyl-*N⁶*-[6-(3-fluoro-phenyl)-pyridin-3-ylmethyl]-9*H*-purine-2,6-diamine, *N*²-(4-amino-cyclohexyl)-9-cyclopentyl-*N*⁶-[6-(2-methoxy-phenyl)-pyridin-3-ylmethyl]-9*H*-purine-2,6-diamine, *N*²-(4-aminocyclohexyl)-9-cyclopentyl-*N*⁶-[6-(2-hydroxy-phenyl)-pyridin-3-ylmethyl]-9*H*-purine-2,6-diamine, *N*²-(4-amino-cyclohexyl)-9-cyclopentyl-*N*⁶-[6-(2-amino-phenyl)-pyridin-3-ylmethyl]-9*H*-purine-2,6-diamine, *N*²-(4-amino-cyclohexyl)-9-cyclopentyl-*N⁶*-(6-pyrazol-1-yl-pyridin-3-ylmethyl)-9*H*-purine-2,6-diamine, 4-(5-{ [2-(4-amino-cyclohexylamino)-9-cyclopentyl-9H-purine-6-ylamino]-methyl}-pyridin-2-yl)-benzoic acid, *N*²-(2-amino-cyclohexyl)-*N⁶*-[2,2']bipyridinyl-5-ylmethyl-9-cyclopentyl-9*H-*purine-2,6-diamine, *N*²-(2-amino-cyclohexyl)-9-cyclopentyl-*N*⁶-(6-thiophen-2-yl-pyridin-3-ylmethyl)-9*H*-purine-2,6-diamine, *N*²-(2-amino-cyclohexyl)-9-cyclopentyl-*N*⁶-(6-thiophen-3-yl-pyridin-3-ylmethyl)-9*H*-purine-2,6-diamine, *N*²-(2-amino-cyclohexyl)-9-cyclopentyl-*N*⁶-(6-furan-2-yl-pyridin-3-ylmethyl)-9*H*-purine-2,6-diamine, *N*²-(2-amino-cyclohexyl)-9-cyclopentyl-*N*⁶-(6-furan-3-yl-pyridin-3-ylmethyl)-9*H*-purine-2,6-diamine, *N*²-(2-amino-cyclohexyl)-9-cyclopentyl-*N⁶*-[6-(3-fluoro-phenyl)-pyridin-3-ylmethyl]-9*H*-purine-2,6-diamine, *N*²-(2-amino-cyclohexyl)-9-cyclopentyl-*N*⁶-[6-(2-methoxy-phenyl)-pyridin-3-ylmethyl]-9*H*-purine-2,6-diamine, *N²*-(2-amino-cyclohexyl)-9-cyclopentyl-*N*⁶-[6-(2-hydroxy-phenyl)-pyridin-3-ylmethyl]-9*H*-purine-2,6-diamine, *N*²-(2-amino-cyclohexyl)-9-cyclopentyl-*N*⁶-[6-(2-aminophenyl)-pyridin-3-ylmethyl]-9*H*-purine-2,6-diamine, *N*²-(2-amino-cyclohexyl)-9-cyclopentyl-*N*⁶-(6-pyrazol-1-yl-pyridin-3-ylmethyl)-9*H*-purine-2,6-diamine, 4-(5-{[2-(2-amino-cyclohexylamino)-9-cyclopentyl-9H-purine-6-ylamino]-methyl}-pyridin-2-yl)-benzoic acid, *N*²-(3-amino-cyclohexyl)-*N*⁶-[2,2']bipyridinyl-5-ylmethyl-9-cyclopentyl-9*H*-purine-2,6-diamine, *N*²-(3-amino-cyclohexyl)-9-cyclopentyl-*N*⁶-(6-thiophen-2-yl-pyridin-3-ylmethyl)-9*H*-purine-2,6-diamine, *N*²-(3-amino-cyclohexyl)-9-cyclopentyl-*N*⁶-(6-thiophen-3-yl-pyridin-3-ylmethyl)-9*H*-purine-2,6-diamine, *N*²-(3-amino-cyclohexyl)-9-cyclopentyl-*N*⁶-(6-furan-2-yl-pyridin-3-ylmethyl)-9*H*-purine-2,6-diamine, *N*²-(3-amino-cyclohexyl)-9-cyclopentyl-*N*⁶-(6-furan-3-yl-pyridin-3-ylmethyl)-9*H*-purine-2,6-diamine, *N*²-(3-amino-cyclohexyl)-9-cyclopentyl-*N*⁶-[6-(3-fluoro-phenyl)-pyridin-3-ylmethyl]-9*H*-purine-2,6-diamine, *N*²-(3-amino-cyclohexyl)-9-cyclopentyl-*N*⁶-[6-(2-methoxy-phenyl)-pyridin-3-ylmethyl]-9*H*-purine-2,6-diamine, *N²*-(3-amino-cyclohexyl)-9-cyclopentyl-*N*⁶-[6-(2-hydroxy-phenyl)-pyridin-3-ylmethyl]-9*H*-purine-2,6-diamine, *N*²-(3-amino-cyclohexyl)-9-cyclopentyl-*N⁶*-[6-(2-aminophenyl)-pyridin-3-ylmethyl]-9*H*-purine-2,6-diamine, *N*²-(3-amino-cyclohexyl)-9-cyclopentyl-*N*⁶-(6-pyrazol-1-yl-pyridin-3-ylmethyl)-9*H*-purine-2,6-diamine, 4-(5-{[2-(3-amino-cyclohexylamino)-9-cyclopentyl-9H-purine-6-ylamino]-methyl}-pyridin-2-yl)-benzoic acid, 4-{9-cyclopentyl-6-[(6-thiophen-2-yl-pyridin-3-ylmethyl)-amino]-9*H-*purine-2-ylamino}-cyclohexanol, 4-{9-cyclopentyl-6-[(6-thiophen-3-yl-pyridin-3-ylmethyl)-amino]-9*H*-purine-2-ylamino}-cyclohexanol, 4-{9-cyclopentyl-6-[(6-furan-2-yl-pyridin-3-ylmethyl)-amino]-9*H*-purine-2-ylamino}-cyclohexanol, 4-{9-cyclopentyl-6-[(6-furan-3-yl-pyridin-3-ylmethyl)-amino]-9*H*-purine-2-ylamino}-cyclohexanol, 4-(9-cyclopentyl-6-{[6-(3-fluoro-phenyl)-pyridin-3-ylmethyl]-amino }-9*H*-purine-2-ylamino)-cyclohexanol, 4-(9-cyclopentyl-6-{[6-(2-methoxy-phenyl)-pyridin-3-ylmethyl]-amino}-9*H*-purine-2-ylamino)-cyclohexanol, 4-(9-cyclopentyl-6-{[6-(2-hydroxy-phenyl)-pyridin-3-ylmethyl]-amino}-9*H*-purine-2-ylamino)-cyclohexanol, 4-(9-cyclopentyl-6-{[6-(2-amino-phenyl)-pyridin-3-ylmethyl]-amino}-9*H*-purine-2-ylamino)-cyclohexanol, 4-{9-cyclopentyl-6-[(6-pyrazol-1-yl-pyridin-3-ylmethyl)-amino]-9*H*-purine-2-ylamino}-cyclohexanol, 4-(5-{[9-cyclopentyl-2-(4-hydroxy-cyclohexylamino)-9H-purine-6-ylamino]-methyl}-pyridin-2-yl)-benzoic acid, 1-{9-cyclopentyl-6-[(6-thiophen-2-yl-pyridin-3-ylmethyl)-amino]-9*H*-purine-2-ylamino}-2-methyl-propan-2-ol, 1-{9-cyclopentyl-6-[(6-thiophen-3-yl-pyridin-3-ylmethyl)-amino]-9*H*-purine-2-ylamino}-2-methyl-propan-2-ol, 1-{9-cyclopentyl-6-[(6-furan-2-yl-pyridin-3-ylmethyl)-amino]-9*H*-purine-2-ylamino}-2-methyl-propan-2-ol, 1-{9-cyclopentyl-6-[(6-furan-3-yl-pyridin-3-ylmethyl)-amino]-9*H*-purine-2-ylamino}-2-methyl-propan-2-ol, 1-(9-cyclopentyl-6-{[6-(3-fluoro-phenyl)-pyridin-3-ylmethyl]-amino}-9*H*-purine-2-ylamino)-2-methyl-propan-2-ol, 1-(9-cyclopentyl-6-{[6-(2-methoxy-phenyl)-pyridin-3-ylmethyl]-amino}-9*H*-purine-2-ylamino)-2-methyl-propan-2-ol, 1-(9-cyclopentyl-6-{[6-(2-hydroxy-phenyl)-pyridin-3-ylmethyl]-amino}-9*H-*purine-2-ylamino)-2-methyl-propan-2-ol, 1-(9-cyclopentyl-6-{[6-(2-amino-phenyl)-pyridin-3-ylmethyl]-amino }-9*H*-purine-2-ylamino)-2-methyl-propan-2-ol, 1-{9-cyclopentyl-6-[(6-pyrazol-1-yl-pyridin-3-ylmethyl)-amino]-9*H*-purine-2-ylamino }-2-methyl-propan-2-ol, 4-(5-{[9-cyclopentyl-2-(2-hydroxy-2-methyl-propylamino)-9*H-*purine-6-ylamino]-methyl}-pyridin-2-yl)-benzoic acid, 4-{9-cyclopentyl-6-[(6-thiophen-2-yl-pyridin-3-ylmethyl)-amino]-9*H*-purine-2-ylamino }-2-methyl-butan-2-ol, 4-{9-cyclopentyl-6-[(6-thiophen-3-yl-pyridin-3-ylmethyl)-amino]-9*H*-purine-2-ylamino}-2-methyl-butan-2-ol, 4-{9-cyclopentyl-6-[(6-furan-2-yl-pyridin-3-ylmethyl)-amino]-9*H*-purine-2-ylamino}-2-methyl-butan-2-ol, 4-{9-cyclopentyl-6-[(6-furan-3-yl-pyridin-3-ylmethyl)-amino]-9*H*-purine-2-ylamino}-2-methyl-butan-2-ol, 4-(9-cyclopentyl-6-{[6-(3-fluoro-phenyl)-pyridin-3-ylmethyl]-amino}-9*H*-purine-2-ylamino)-2-methyl-butan-2-ol, 4-(9-cyclopentyl-6-{[6-(2-methoxy-phenyl)-pyridin-3-ylmethyl]-amino}-9*H*-purine-2-ylamino)-2-methyl-butan-2-ol, 4-(9-cyclopentyl-6-{[6-(2-hydroxy-phenyl)-pyridin-3-ylmethyl]-amino}-9*H*-purine-2-ylamino)-2-methyl-butan-2-ol, 4-(9-cyclopentyl-6-{[6-(2-amino-phenyl)-pyridin-3-ylmethyl]-amino}-9*H-*purine-2-ylamino)-2-methyl-butan-2-ol, 4-{9-cyclopentyl-6-[(6-pyrazol-1-yl-pyridin-3-ylmethyl)-amino]-9*H*-purine-2-ylamino}-2-methyl-butan-2-ol, 4-(5-{[9-cyclopentyl-2-(3-hydroxy-3-methyl-butylamino)-9*H*-purine-6-ylamino]-methyl}-pyridin-2-yl)-benzoic acid, *N*²-(2-amino-propyl)-*N*⁶-[2,2']bipyridinyl-5-ylmethyl-9-cyclopentyl-9*H*-purine-2,6-diamine, *N*²-(2-amino-propyl)-9-cyclopentyl-*N*⁶-(6-thiophen-2-yl-pyridin-3-ylmethyl)-9*H*-purine-2,6-diamine, *N*²-(2-amino-propyl)-9-cyclopentyl-*N*⁶-(6-thiophen-3-yl-pyridin-3-ylmethyl)-9*H*-purine-2,6-diamine, *N*²-(2-amino-propyl)-9-cyclopentyl-*N*⁶-(6-furan-2-yl-pyridin-3-ylmethyl)-9*H*-purine-2,6-diamine, *N*²-(2-amino-propyl)-9-cyclopentyl-*N*⁶-(6-furan-3-yl-pyridin-3-ylmethyl)-9*H*-purine-2,6-diamine, *N*²-(2-amino-propyl)-9-cyclopentyl-*N⁶*-[6-(3-fluoro-phenyl)-pyridin-3-ylmethyl]-9*H*-purine-2,6-diamine, *N*²-(2-amino-propyl)-9-cyclopentyl-*N*⁶-[6-(2-methoxy-phenyl)-pyridin-3-ylmethyl]-9*H*-purine-2,6-diamine, *N*²-(2-amino-propyl)-9-cyclopentyl-*N*⁶-[6-(2-hydroxy-phenyl)-pyridin-3-ylmethyl]-9*H*-purine-2,6-diamine, *N*²-(2-amino-propyl)-9-cyclopentyl-*N*⁶-[6-(2-amino-phenyl)-pyridin-3-ylmethyl]-9*H*-purine-2,6-diamine, *N*²-(2-amino-propyl)-9-cyclopentyl-*N*⁶-(6-pyrazol-1-yl-pyridin-3-ylmethyl)-9*H*-purine-2,6-diamine, 4-(5-{[2-(2-amino-propyl)-9-cyclopentyl-9H-purine-6-ylamino]-methyl}-pyridin-2-yl)-benzoic acid, *N*²-(3-amino-propyl)-*N*⁶-[2,2']bipyridinyl-5-ylmethyl-9-cyclopentyl-9*H*-purine-2,6-diamine, *N*²-(3-amino-propyl)-9-cyclopentyl-*N*⁶-(6-thiophen-2-yl-pyridin-3-ylmethyl)-9*H*-purine-2,6-diamine, *N*²-(3-amino-propyl)-9-cyclopentyl-*N*⁶-(6-thiophen-3-yl-pyridin-3-ylmethyl)-9*H*-purine-2,6-diamine, *N*²-(3-amino-propyl)-9-cyclopentyl-*N*⁶-(6-furan-2-yl-pyridin-3-ylmethyl)-9*H*-purine-2, 6-diamine, *N*²-(3-amino-propyl)-9-cyclopentyl-*N*⁶-(6-furan-3-yl-pyridin-3-ylmethyl)-9*H-*purine-2,6-diamine, *N*²-(3-amino-propyl)-9-cyclopentyl-*N*⁶-[6-(3-fluoro-phenyl)-pyridin-3-ylmethyl]-9*H*-purine-2,6-diamine, *N*²-(3-amino-propyl)-9-cyclopentyl-*N*⁶-[6-(2-methoxy-phenyl)-pyridin-3-ylmethyl]-9*H*-purine-2,6-diamine, *N*²-(3-amino-propyl)-9-cyclopentyl-*N*⁶-[6-(2-hydroxy-phenyl)-pyridin-3-ylmethyl]-9*H*-purine-2,6-diamine, *N*²-(3-amino-propyl)-9-cyclopentyl-*N*⁶-[6-(2-amino-phenyl)-pyridin-3-ylmethyl]-9*H-*purine-2,6-diamine, *N*²-(3-amino-propyl)-9-cyclopentyl-*N*⁶-(6-pyrazol-1-yl-pyridin-3-ylmethyl)-9*H*-purine-2,6-diamine, 4-(5-{[2-(3-amino-propyl)-9-cyclopentyl-9H-purine-6-ylamino]-methyl{-pyridin-2-yl)-benzoic acid, *N*²-(2-amino-ethyl)-*N*⁶-[2,2']bipyridinyl-5-ylmethyl-9-cyclopentyl-9*H*-purine-2,6-diamine, *N*²-(2-amino-ethyl)-9-cyclopentyl-*N⁶*-(6-thiophen-2-yl-pyridin-3-ylmethyl)-9*H*-purine-2,6-diamine, *N*²-(2-amino-ethyl)-9-cyclopentyl-*N*⁶-(6-thiophen-3-yl-pyridin-3-ylmethyl)-9*H*-purine-2,6-diamine, *N*²-(2-amino-ethyl)-9-cyclopentyl-*N*⁶-(6-furan-2-yl-pyridin-3-ylmethyl)-9*H-*purine-2,6-diamine, *N*²-(2-amino-ethyl)-9-cyclopentyl-*N⁶*-(6-furan-3-yl-pyridin-3-ylmethyl)-9*H*-purine-2,6-diamine, *N*²-(2-amino-ethyl)-9-cyclopentyl-*N*⁶-[6-(3-fluoro-phenyl)-pyridin-3-ylmethyl]-9*H*-purine-2,6-diamine, *N*²-(2-amino-ethyl)-9-cyclopentyl-*N*⁶-[6-(2-methoxy-phenyl)-pyridin-3-ylmethyl]-9*H*-purine-2,6-diamine, *N*²-(2-amino-ethyl)-9-cyclopentyl-*N*⁶-[6-(2-hydroxy-phenyl)-pyridin-3-ylmethyl]-9*H*-purine-2,6-diamine, *N*²-(2-amino-ethyl)-9-cyclopentyl-*N*⁶-[6-(2-amino-phenyl)-pyridin-3-ylmethyl]-9*H*-purine-2,6-diamine, *N*²-(2-amino-ethyl)-9-cyclopentyl-*N*⁶-(6-pyrazol-1-yl-pyridin-3-ylmethyl)-9*H*-purine-2,6-diamine, 4-(5-{[2-(2-amino-ethyl)-9-cyclopentyl-9H-purine-6-ylamino]-methyl}-pyridin-2-yl)-benzoic acid, 3-{9-cyclopentyl-6-[(6-thiophen-2-yl-pyridin-3-ylmethyl)-amino]-9*H*-purine-2-ylamino}-propan-1-ol, 3-{9-cyclopentyl-6-[(6-thiophen-3-yl-pyridin-3-ylmethyl)-amino]-9*H*-purine-2-ylamino}-propan-1-ol, 3-{9-cyclopentyl-6-[(6-furan-2-yl-pyridin-3-ylmethyl)-amino]-9*H*-purine-2-ylamino}-propan-1-ol, 3-{9-cyclopentyl-6-[(6-furan-3-yl-pyridin-3-ylmethyl)-amino]-9*H*-purine-2-ylamino}-propan-1-ol, 3-(9-cyclopentyl-6-{[6-(3-fluoro-phenyl)-pyridin-3-ylmethyl]-amino}-9*H*-purine-2-ylamino)-propan-1-ol, 3-(9-cyclopentyl-6-{[6-(2-methoxy-phenyl)-pyridin-3-ylmethyl]-amino}-9*H*-purine-2-ylamino)-propan-1-ol, 3-(9-cyclopentyl-6-{[6-(2-hydroxy-phenyl)-pyridin-3-ylmethyl]-amino}-9*H*-purine-2-ylamino)-propan-1-ol, 3-(9-cyclopentyl-6-{[6-(2-amino-phenyl)-pyridin-3-ylmethyl]-amino}-9*H*-purine-2-ylamino)-propan-1-ol, 3-{9-cyclopentyl-6-[(6-pyrazol-1-yl-pyridin-3-ylmethyl)-amino]-9*H*-purine-2-ylamino}-propan-1-ol, 4-(5-{[9-cyclopentyl-2-(3-hydroxy-propylamino)-9*H*-purine-6-ylamino]-methyl}-pyridin-2-yl)-benzoic acid, (*R*)-3-{9-cyclopentyl-6-[(6-thiophen-2-yl-pyridin-3-ylmethyl)-amino]-9*H*-purine-2-ylamino}-pentan-2-ol, (*R*)-3-{9-cyclopentyl-6-[(6-thiophen-3-yl-pyridin-3-ylmethyl)-amino]-9*H*-purine-2-ylamino}-pentan-2-ol, (*R*)-3-{9-cyclopentyl-6-[(6-furan-2-yl-pyridin-3-ylmethyl)-amino]-9*H*-purine-2-ylamino}-pentan-2-ol, (*R*)-3-{9-cyclopentyl-6-[(6-furan-3-yl-pyridin-3-ylmethyl)-amino]-9*H*-purine-2-ylamino}-pentan-2-ol, (*R*)-3-(9-cyclopentyl-6-{[6-(3-fluoro-phenyl)-pyridin-3-ylmethyl]-amino}-9H-purine-2-ylamino)-pentan-2-ol, (*R*)-3-(9-cyclopentyl-6-{[6-(2-methoxy-phenyl)-pyridin-3-ylmethyl]-amino}-9H-purine-2-ylamino)-pentan-2-ol, (*R*)-3-(9-cyclopentyl-6-{[6-(2-hydroxy-phenyl)-pyridin-3-ylmethyl]-amino}-9H-purine-2-ylamino)-pentan-2-ol, *(R)-3-*(9-cyclopentyl-6-{[6-(2-amino-phenyl)-pyridin-3-ylmethyl]-amino}-9H-purine-2-ylamino)-pentan-2-ol, (*R*)-3-{9-Cyclopentyl-6-[(6-pyrazol-1-yl-pyridin-3-ylmethyl)-amino]-9*H*-purine-2-ylamino}-pentan-2-ol, 4-(5-{[9-cyclopentyl-2-((*R*)-1-ethyl-2-hydroxy-propylamino)-9*H*-purine-6-ylamino]-methyl}-pyridin-2-yl)-benzoic acid, (*R*)-2-{9-cyclopentyl-6-[(6-thiophen-2-yl-pyridin-3-ylmethyl)-amino]-9*H*-purine-2-ylamino}-butan-1-ol, (*R*)-2-{9-cyclopentyl-6-[(6-thiophen-3-yl-pyridin-3-ylmethyl)-amino]-9*H*-purine-2-ylamino}-butan-1-ol, (*R*)-2-{9-cyclopentyl-6-[(6-furan-2-yl-pyridin-3-ylmethyl)-amino]-9*H*-purine-2-ylamino}-butan-1-ol, (*R*)-2-{9-cyclopentyl-6-[(6-furan-3-yl-pyridin-3-ylmethyl)-amino]-9*H*-purine-2-ylamino}-butan-1-ol, (*R*)-2-(9-cyclopentyl-6-{[6-(3-fluoro-phenyl)-pyridin-3-ylmethyl]-amino}-9*H*-purine-2-ylamino)-butan-1-ol, (*R*)-2-(9-cyclopentyl-6-{[6-(2-methoxy-phenyl)-pyridin-3-ylmethyl]-amino}-9*H*-purine-2-ylamino)-butan-1-ol, (*R*)-2-(9-cyclopentyl-6-{[6-(2-hydroxy-phenyl)-pyridin-3-ylmethyl]-amino}-9*H*-purine-2-ylamino)-butan-1-ol, (*R*)-2-(9-cyclopentyl-6-{[6-(2-amino-phenyl)-pyridin-3-ylmethyl]-amino}-9*H*-purine-2-ylamino)-butan-1-ol, (*R*)-2-{9-cyclopentyl-6-[(6-pyrazol-1-yl-pyridin-3-ylmethyl)-amino]-9*H*-purine-2-ylamino}-butan-1-ol, 4-(5-{[9-cyclopentyl-2-((*R*)-1-hydroxymethyl-propylamino)-9*H*-purine-6-ylamino]-methyl}-pyridin-2-yl)-benzoic acid.

### GENERAL SYNTHETIC PROCEDURES

The compounds of the present invention were prepared by conventional chemical procedures which allowed high variability of substituents in positions 2 and 6 of the purine moiety. Suitable synthetical approaches are shown in Scheme 1.

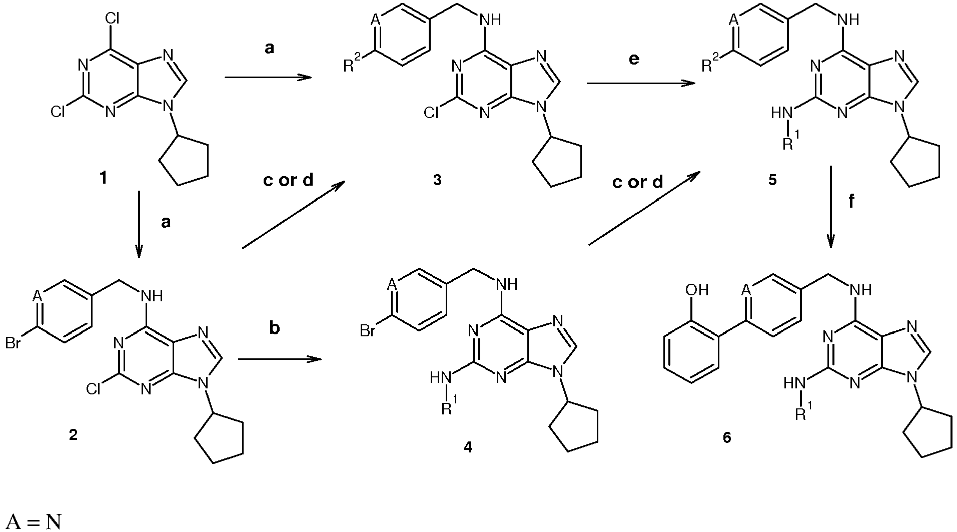

### Reagents and conditions:

**a**: appropriate amine, DIPEA, *n*-propanol, 120°C (sealed tube), 4-8 hours
**b**: *trans*-1,4-diaminocyclohexane, 160 °C (sealed tube), 4 hours
**c**: appropriate arylboronic acid, Pd(OAc)₂, K₃PO₄, TBAB, DMF, 80-120°C, 4-48 hours
**d**: appropriate arylboronic acid, Pd(dba)₂, PPh₃, Na₂CO₃, DME, water, 80°C, 8-16 hours
**e**: appropriate amine, DIPEA, NMP, 160°C, 4-72 hours
**f**: 1. BBr₃, DCM, 2. methanol

The synthesis starts from commercially available 2,6-dichloropurine, which was in the first step alkylated by cyclopentanol *via* Mitsunobu alkylation to obtain 9-cyclopentyl-2,6-dichloro-9H-purine (1) which is then reacted with the appropriate 4-bromobenzylamine or C-(6-bromo-pyridin-3-yl)methylamine to obtain the compound of structure (2). In some cases, the reaction with appropriate 1-[6-(subst.phenyl)pyridin-3-yl]methanamine or 1-[6-(heteroaryl)pyridin-3-yl]methanamine is performed to obtain compound (3). The substitution of the chlorine atom in position 2 of the purine moiety proceeds for compounds (2) or (3) with a large excess of the appropriate amine in the presence of a strong base at the temperature of 160°C to obtain compound (4) or (5). The Suzuki coupling of compounds (2) or (4) with the appropriate aryl or heteroaryl boronic acid proceeds smoothly even in the presence of the chlorine atom in case of compounds (2), (3) or (5). The demethylation of compound (6), wherein R² is 2-methoxyphenyl group, is performed using boron tribromide in dichloromethane under mild conditions.

### PHARMACEUTICAL COMPOSITIONS

The therapeutic compositions comprise about 1% to about 95% of the active ingredient, single-dose forms of administration preferably comprising about 20% to about 90% of the active ingredient, and administration forms which are not single-dose preferably comprising about 5% to about 20% of the active ingredient. Unit dose forms may be, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical compositions of the present invention are prepared in a known manner, for example by means of conventional mixing, granulating, coating, dissolving or lyophilizing processes.

Preferably, solutions of the active ingredient, and in addition also suspensions or dispersions, especially isotonic aqueous solutions, dispersions or suspensions, are used, if being possible for these to be prepared before use, for example in the case of lyophilised compositions which comprise the active substance by itself or together with a carrier, for example mannitol. The pharmaceutical compositions can be sterilised and/or comprise excipients, for example preservatives, stabilisers, wetting agents and/or emulsifiers, solubilizing agents, salts for regulating the osmotic pressure and/or buffers, and they are prepared in a manner known per se, for example by means of conventional dissolving or lyophilising processes. The solutions or suspensions mentioned can comprise viscosity-increasing substances, such as sodium carboxymethylcellulose, dextran, polyvinylpyrrolidone or gelatine.

Suspensions in oil comprise, as the oily component, the vegetable, synthetic or semisynthetic oils customary for injection purposes. Oils which may be mentioned are, in particular, liquid fatty acid esters which contain, as the acid component, a long-chain fatty acid having 8-22, in particular 12-22, carbon atoms, for example lauric acid, tridecylic acid, myristic acid, pentadecylic acid, palmitic acid, margaric acid, stearic acid, arachidonic acid, behenic acid or corresponding unsaturated acids, for example oleic acid, elaidic acid, erucic acid, brasidic acid or linoleic acid, if appropriate with the addition of antioxidants, for example vitamin E, β-carotene or 3,5-di-*tert*-butyl-4-hydroxytoluene. The alcohol component of these fatty acid esters has not more than 6 carbon atoms and is mono- or polyhydric, for example mono-, di- or trihydric alcohol, for example methanol, ethanol, propanol, butanol, or pentanol, or isomers thereof, but in particular glycol and glycerol. Fatty acid esters are, for example: ethyl oleate, isopropyl myristate, isopropyl palmitate, "Labrafil M 2375" (polyoxyethylene glycerol trioleate from Gattefoseé, Paris), "Labrafil M 1944 CS" (unsaturated polyglycolated glycerides prepared by an alcoholysis of apricot kernel oil and made up of glycerides and polyethylene glycol esters; from Gattefoseé, Paris), "Labrasol" (saturated polyglycolated glycerides prepared by an alcoholysis of TCM and made up of glycerides and polyethylene glycol esters; from Gattefoseé, Paris) and/or "Miglyol 812" (triglyceride of saturated fatty acids of chain length C₈ to C₁₂ from Hüls AG, Germany), and in particular vegetable oils, such as cottonseed oil, almond oil, olive oil, castor oil, sesame oil, soybean oil and, in particular, groundnut oil.

The preparation of the injection compositions is carried out in the customary manner under sterile conditions, as are bottling, for example into ampoules or vials, and closing of the containers.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients.

Suitable carriers are, in particular, fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidine, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate. Dyes or pigments can be admixed to the tablets or coated tablet coatings, for example for identification or characterisation of different doses of active ingredient.

Pharmaceutical compositions, which can be used orally, are also hard capsules of gelatine and soft, closed capsules of gelatine and a plasticiser, such as glycerol or sorbitol. The hard capsules can contain the active ingredient in the form of granules, mixed for example with fillers, such as maize starch, binders and/or lubricants, such as talc or magnesium stearate, and stabilisers if appropriate. In soft capsules, the active ingredient is preferably dissolved or suspended in suitable liquid excipients, such as greasy oils, paraffin oil or liquid polyethylene glycol or fatty acid esters of ethylene glycol or propylene glycol, it being likewise possible to add stabilisers and detergents, for example of the polyethylene sorbitan fatty acid ester type.

Other oral forms of administration are, for example, syrups prepared in the customary manner, which comprise the active ingredient, for example, in suspended form and in a concentration of about 5% to 20%, preferably about 10% or in a similar concentration which results in a suitable individual dose, for example, when 5 or 10 ml are measured out. Other forms are, for example, also pulverulent or liquid concentrates for preparing of shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols. Compositions which are suitable for parental administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and, if appropriate, stabilizers. The active ingredient can also be present here in the form of a lyophilisate, if appropriate, together with excipients, and be dissolved before parenteral administration by addition of suitable solvents. Solutions such as are used, for example, for parental administration can also be used as infusion solutions. Preferred preservatives are, for example, antioxidants, such as ascorbic acid, or microbicides, such as sorbic or benzoic acid.

Ointments are oil-in-water emulsions which comprise not more than 70%, preferably 20 - 50% of water or aqueous phase. The fatty phase consists, in particular, hydrocarbons, for example vaseline, paraffin oil or hard paraffins, which preferably comprise suitable hydroxy compounds, such as fatty alcohols or esters thereof, for example cetyl alcohol, or wool wax alcohols, such as wool wax, to improve the water-binding capacity. Emulsifiers are corresponding lipophilic substances, such as sorbitan fatty acid esters (Spans), for example sorbitan oleate and/or sorbitan isostearate. Additives to the aqueous phase are, for example, humectants, such as polyalcohols, for example glycerol, propylene glycol, sorbitol and/or polyethylene glycol, or preservatives and odoriferous substances.

Tinctures and solutions usually comprise an aqueous-ethanolic base to which, humectants for reducing evaporation, such as polyalcohols, for example glycerol, glycols and/or polyethylene glycol, and re-oiling substances, such as fatty acid esters with lower polyethylene glycols, i.e. lipophilic substances soluble in the aqueous mixture to substitute the fatty substances removed from the skin with ethanol, and, if necessary, other excipients and additives, are admixed.

The invention also relates to a process or method for treatment of the disease states mentioned above. The compounds can be administered prophylactically or therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition. A daily dose of about 0.1 to about 5 g, preferably 0.5 g to about 2 g, of a compound of the present invention is administered here for a body weight of about 70 kg.

### Brief Description of Drawings

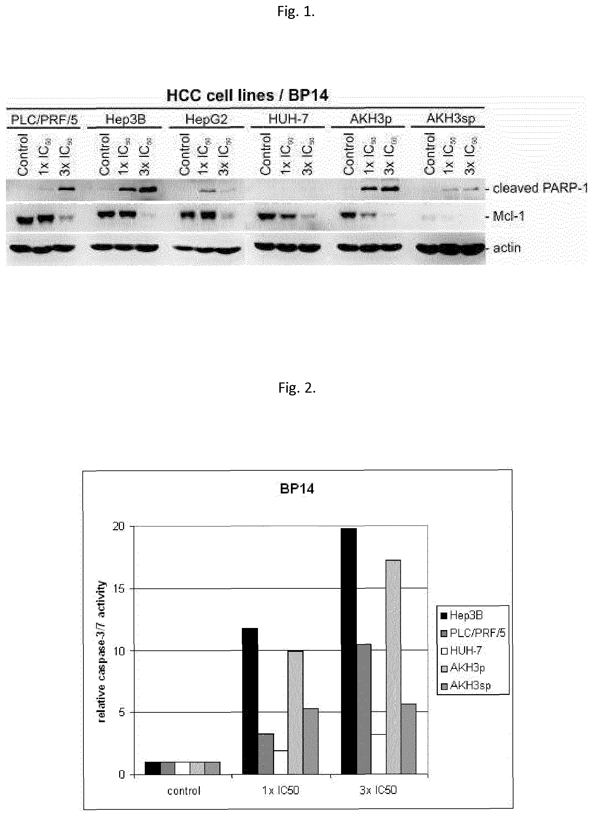

Figure 1 shows induction of apoptosis in different hepatocellular carcinoma cell lines treated with compound BP14. Asynchronous cells were exposed for 24 hours to the indicated concentrations of BP14 and then protein levels of cleaved PARP-1 and antiapoptotic protein Mcl-1 were analyzed by immunoblotting. Level of actin was detected to verify equal protein loading.
Figure 2 shows induction of apoptosis in different hepatocellular carcinoma cell lines treated with compound BP14. The activities of caspases-3/7 were measured using a fluorogenic substrate Ac-DEVD-AMC in lysates of cells treated with increasing doses of compound BP14.

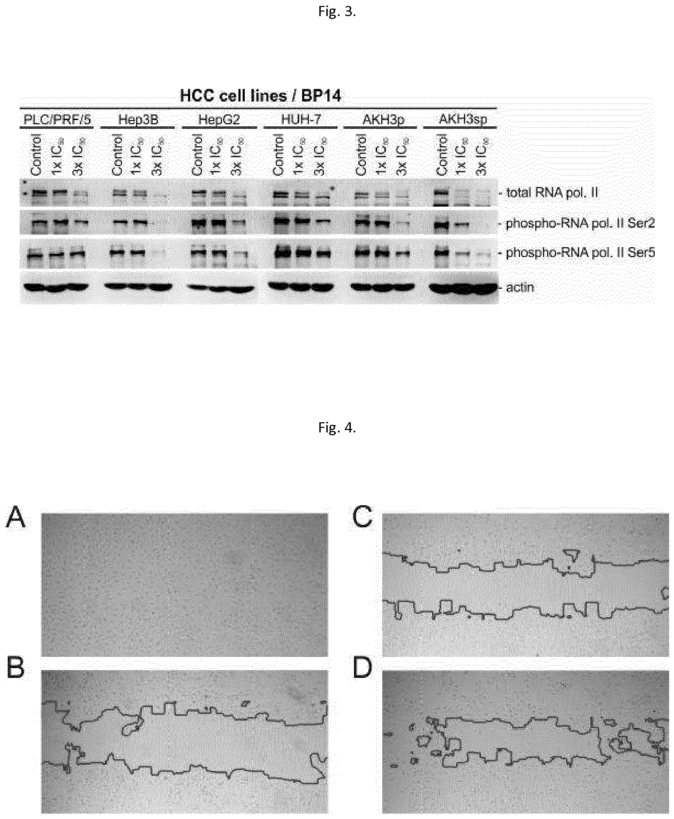

Figure 3 shows immunoblot analysis of inhibition of transcription in different hepatocellular carcinoma cell lines treated with compound BP14 for 24 hours. Actin levels were detected to verify equal protein loading.
Figure 4 shows the effect of 2-substituted-6-biarylmethylamino-9-cyclopentyl-9*H-*purines on migration of human umbilical vein endothelial cells (HUVECs). Determination of migration using "*in house*" software calculated as the proportion of pixels in the image that were not covered by cells. (A) control cells; (B) positive control (the cells were kept in a serum-free medium), (C) cells treated by BP30 (100 nM); (D) cells treated by BP36 (100 nM).

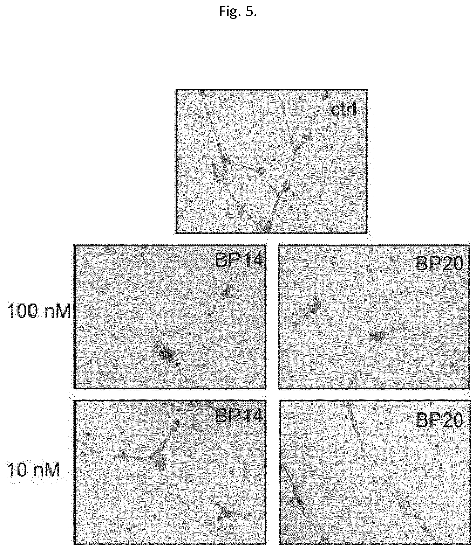

Figure 5 shows anti-angiogenic activity of BP14 and BP20. For tube formation assays, HUVECs were seeded on Matrigel-coated dishes in the presence of the indicated doses of BP14 adn BP20 and incubated for 24 h to allow formation of a capillary network.

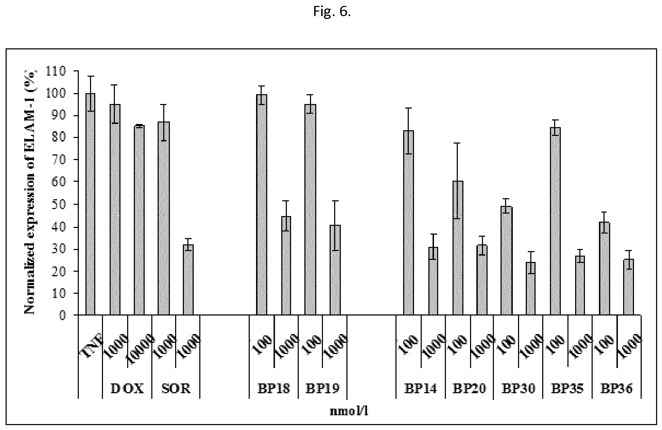

Figure 6 shows an expression of ELAM-1 by HUVECs co-cultured with different doses (in nanomolar concentration) of the tested inhibitors for 4 hours. Data are presented as the mean and standard deviation of three different experiments.

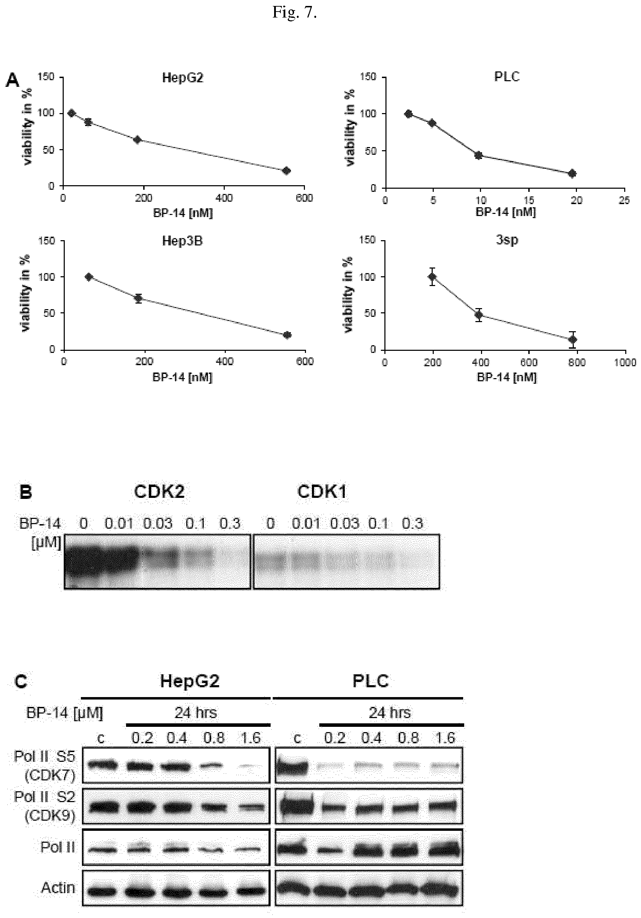

Figure 7 shows that BP-14 decreases cell viability of hepatoma cells and blocks multiple CDKs. A, dose-dependent effect of BA-12 on the viability of human HepG2, PLC, Hep3B and 3sp hepatoma cells. B, inhibition of CDK1 and CDK2 activity by BP-14 in cell-free extracts. C, suppression of CDK7 and CDK9 activity after exposure to different concentrations of BP-14 for 24 hours in HepG2 and PLC cells. As detected by immunoblotting, CDK7 and CDK9 activities correspond to serine 5 and serine 2 phosphorylation of RNA polymerase II, respectively. The expression of actin indicates equal loading of protein samples. c, control (untreated cells). Error bars depict SD from at least three individual experiments.

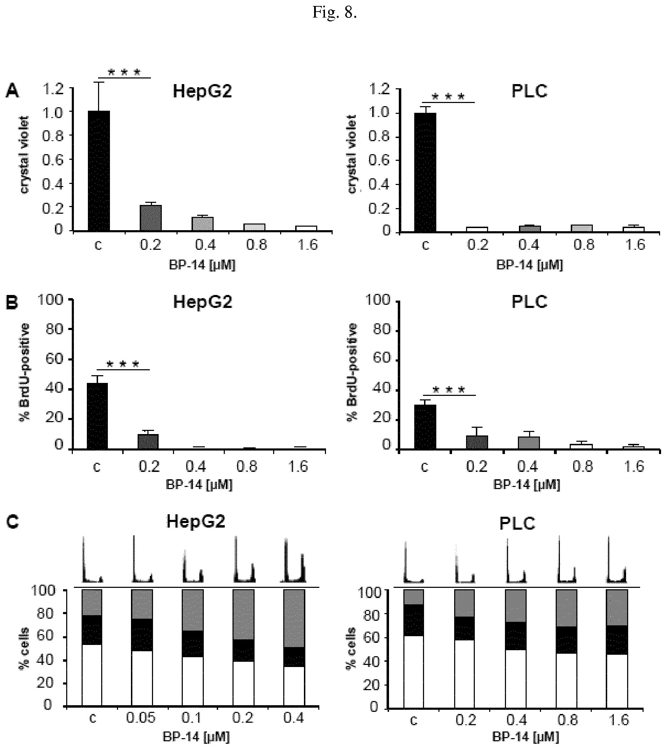

Figure 8 shows that BP-14 interferes with clonogenicity and cell cycle progression of HCC cells. A, quantitative evaluation of crystal violet-positive colonies generated by HepG2 (left panel) and PLC cells (right panel). Cells with pretreated with different concentrations of BP-14. B, HepG2 (left) and PLC cells (right) were exposed to BP-14 for 24 hours and the DNA synthesis analyzed by BrdU incorporation. C, flow cytometry showing the cell cycle distribution of HepG2 (left) and PLC cells (right) after treatment with different concentrations of BP-14 for 24 hours. The cellular DNA content is shown in histograms (upper panel) and the percentages of cells in G1, S or G2 phase are depicted in bars after quantification (lower panel). c, control (untreated cells). Error bars depict SD from at least three individual experiments. Statistical significance is indicated with asterisks (*** p<0.005).

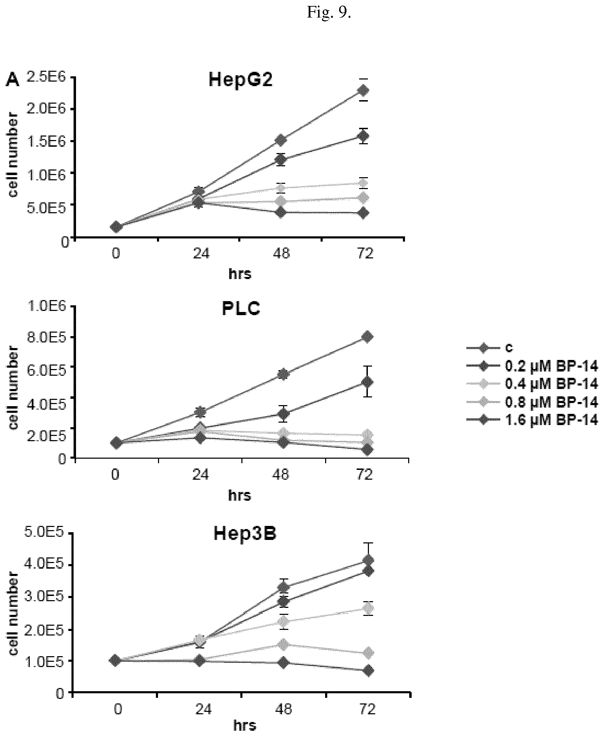

Figure 9 displays proliferation of HCC cells after exposure to BP-14. Proliferation kinetics of HepG2, PLC and Hep3B cells cells after treatment with different concentrations of BP-14. Error bars depict SD from at least three individual experiments.

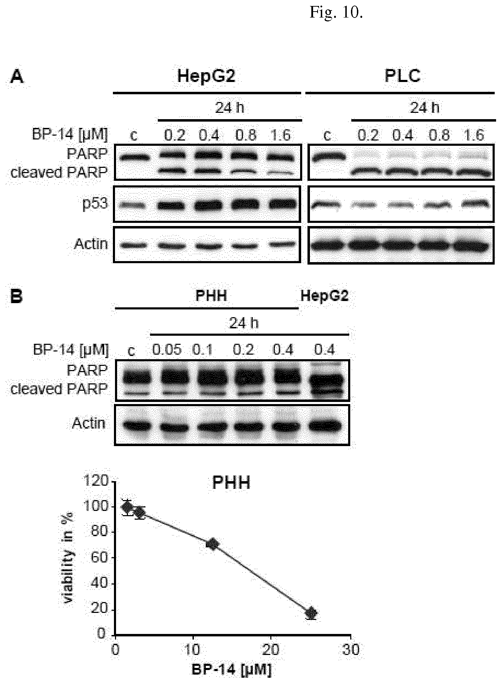

Figure 10 displays apoptosis induced by BP-14 in HCC cells but not in primary human hepatocytes (PHHs). A, cleavage of PARP after treatment of HepG2 and PLC cells with different concentrations of BP-14 for 24 hours. B, PARP cleavage (upper panel) and determination of dose-dependent effects of BP-14 on the viability (lower panel) of PHHs. PARP cleavage of HepG2 cells are included as positive control. Actin is shown as loading control. c, control (untreated cells). Error bars depict SD from at least three individual experiments.

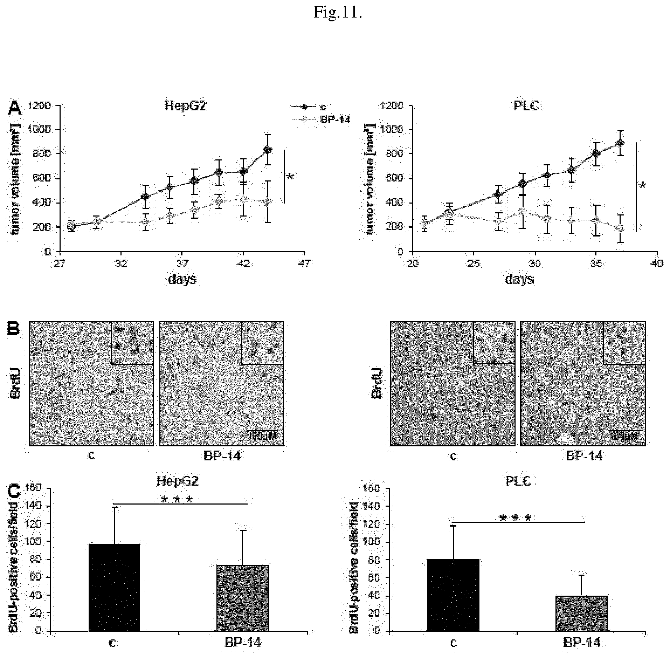

Figure 11 shows intervention of xenografted HCC models with BP-14. Tumors were generated by subcutaneous injections of HepG2 and PLC cells into immunodeficient SCID mice. Pharmacological intervention was performed in tumor-bearing mice by daily intraperitoneal injection of BP-14 for 17 days. A, volumes of HepG2- and PLC-derived tumors in the absence of compounds (control) and after interference with BP-14. B, immunohistochemistry showing tumor sections stained anti-BrdU antibody. Inserts show BrdU labeling at higher magnification. C, quantitative analysis of BrdU incorporation. c, control (untreated cells). Error bars depict SD from three individual experiments that were performed in quadruplicates. Statistical significance is indicated with asterisks (* p<0.05, *** p<0.005).

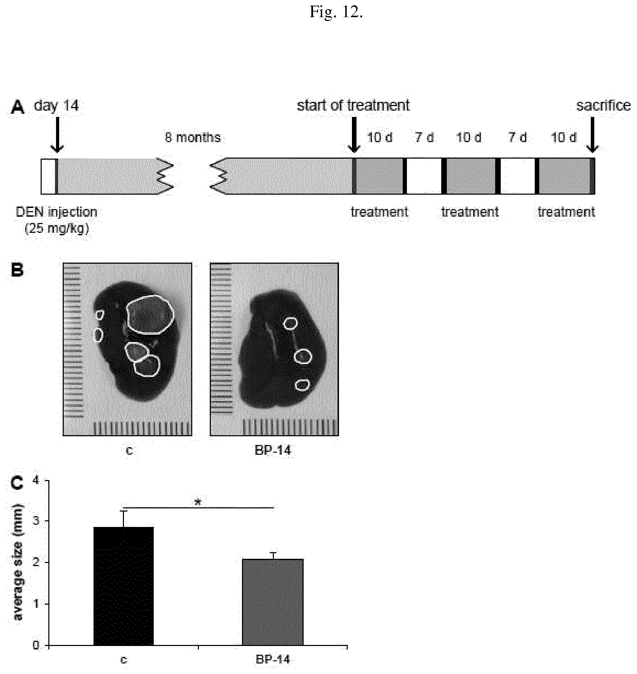

Figure 12 shows that BP-14 reduces DEN-induced hepatoma formation. Endogenous liver cancer was induced by a single DEN injection in 14 days-old C57BL/6J mice. A, scheme depicting the treatment schedule with BP-14. After 8 month (hatched box), DEN-induced mice were subjected to 3 cycles of drug treatment for 10 days (green boxes) and a release from Bp-14 for 7 days between the cycles. B, representative morphologies of DEN-induced hepatoma (control) and those treated with BP-14. White circles indicate cancerous liver nodules. C, the diameters of cancerous nodules were scored on the surface of livers and depicted in bars. Statistical significance is indicated with asterisks (*, p<0.05).

### Examples of Carrying Out the Invention

The following examples serve to illustrate the invention.

The starting materials for the compounds of the formula **I** is commercially available (Sigma-Aldrich, Fluka, etc.).

Melting points were determined on a Boetius stage and are corrected. ¹H NMR spectra were measured in CDCl₃ or in DMSO-d₆ at 300 K on a Bruker Avance 300 NMR spectrometer (300 MHz) with TMS as an internal standard; chemical shifts are reported in ppm, and coupling constants in Hz. Mass spectra were recorded by using an LCQ ion trap mass spectrometer (Finnigan MAT, San Jose, CA, USA). Merck silica gel Kieselgel 60 (230-400 mesh) was used for column chromatography. Elemental analyses were performed by using an EA 1108 Elemental Analyzer (Fison Instruments); their values (C, H, N) agreed with the calculated ones within acceptable limits. Quadrupole mass spectra were measured on a Micromass ZMD detector with electrospray ionization.

The starting 2,6-dichloro-9-cyclopentylpurine was prepared by a Mitsunobu alkylation method from 2,6-dichloropurine and cyclopentanol.
1) Shum et al. Nucleos. Nucleot. 20, 2001: 1067 - 1078
2) Dreyer et al. J. Med. Chem. 44, 2001: 524 - 530

### Preparation of 9-cyclopentyl-2,6-dichloro-9H-purine.

2,6-Dichloro-9*H*-purine (30.0 mmol), cyclopentanol (60.0 mmol) and triphenylphosphine (36.0 mmol) were dissolved in dry tetrahydrofuran (120 ml) and cooled to 0°C. To the stirred solution diisopropyl azodicarboxylate (36.0 mmol) was added dropwise under an argon atmosphere so that the temperature was kept between 0 and 20°C. The reaction mixture was stirred under an argon atmosphere at 20°C for further 2 hours. The reaction mixture was then evaporated under reduced pressure and the residue was dissolved in boiling toluene (100 ml). After cooling to room temperature the solution was inoculated with small amount of triphenylphosphine oxide and the solution was kept at 5°C for 24 hours. The triphenylphospine oxide was filtered off and the filtrate was evaporated under reduced pressure. The residue was crystallized from ethanol to obtain pure 9-cyclopentyl-2,6-dichloro-9*H*-purine. Yield: 56%, mp: 118-120 °C. Elemental analysis: Calcd.for C₁₀H₁₀Cl₂N₄ (257.12): C, 46.71; H, 3.92; N, 21.79. Found: C, 46.95; H, 3.81; N, 21.70. HPLC-MS (ESI+): 288.10 (99.6%). ¹H NMR (DMSO-d₆): 1.64-1.69(m, 2H), 1.81-1.96(m, 4H), 2.09-2.15(m, 2H), 4.92(qui, J=7.53, 1H, CH), 8.82(s, 1H, CH).

### Preparation of C-(6-bromo-pyridin-3-yl)methylamine

2-Bromo-5-methyl-pyridine (70.0 mmol) and N-bromosuccinimide (80.0 mmol) were dissolved in 1,2-dichloroethane (150 ml) and to this mixture 2,2'-azobis(2-ethylpropionitrile) (1.50 mmol) was added. The reaction mixture was heated under reflux at 85 °C for 15 minutes and next portion of 2,2'-azobis(2-methylpropionitrile) (1.50 mmol) was added and reaction mixture was heated at 85°C for further 15 minutes. After cooling to room temperature was the reaction mixture kept at 5°C for 2 hours and the precipitate was filtered off and washed with smal amount of 1,2-dichloroethane. The filtrate was evaporated under reduced pressure and the crude product was used for further reaction step without purification. The crude 2-bromo-5-bromomethyl-pyridine was dissolved in chloroform (100 ml) and urotropine (70.0 mmol) was added. The reaction mixture was stirred at room temperature for 16 hours. The precipitate was filtered off, washed with small amount of chloroform and dried on air. The crude urotropine salt was refluxed in a mixture of conc.ammonium hydroxide (12 ml) and water (80 ml) for 90 minutes and after cooling to room temperature, 40% formaldehyde (5.0 ml) was added with stirring. The precipitate was filtered off, washed with ice-cold water and dried in vacuum dessicator. The crude product was crystallized from ethanol. Yield: 40% m.p. 105-106°C. Elemental analysis: Calcd.for C₆H₇BrN₂ (187.04): C, 38.53; H, 3.77; N, 14.98. Found: C, 38.22; H, 3.72; N, 14.71. HPLC-MS (ESI+): 188.02 (97.2%). ¹H NMR (DMSO_d₆): 4.04(t, J=5.67, 2H, CH₂), 7.71(d, J=8.19, 1H, ArH), 7.95(dd, J=8.19, J'=1.95, 1H, ArH), 8.51(d, J=1.95, 1H, ArH), 8.74(s(br), 2H, NH₂).

### EXAMPLE 1 (6-bromo-pyridin-3-ylmethyl)-(2-chloro-9-cyclopentyl-9H-purine-6-yl)-amine

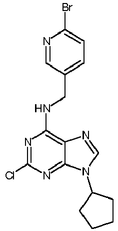

To the suspension of 9-cyclopentyl-2,6-dichloro-9*H*-purine (13.6 mmol) in a mixture of n-propanol (60 ml) and *N*,*N*-diisopropyl-*N*-ethylamine (60.0 mmol) C-(6-bromo-pyridin-3-yl)methylamine (15.0 mmol) was added. The suspension was heated with stirring in a sealed tube under an argon atmosphere at the temperature 120°C for 4 hours. After cooling to room temperature the reaction mixture was left to stand at 5°C overnight and the white solid was filtered off and washed with small amount of ice-cooled isopropanol. The crude product was dried at 80°C for 2 hours and finally crystallized from ethanol. Yield: 71%, m.p.: 178-179 °C. Elemental analysis: Calcd.for C₁₆H₁₆ClBrN₆ (407.70): C, 47.14; H, 3.96; N, 20.61. Found: C, 47.35; H, 3.88; N, 20.48. HPLC-MS (ESI+): 409 (98.5%). ¹H NMR (DMSO₋d₆): 1.64-1.69(m, 2H), 1.81-1.96(m, 4H), 2.09-2.15(m, 2H), 4.61(s(br), 2H, CH2), 4.77(qui, J=7.20, 1H, CH), 7.59(d, J=8.19, 1H, ArH), 7.70(d, J=8.19, 1H, ArH), 8.26(s, 1H, CH), 8.38(s, 1H, ArH), 8.82(s(br), 1H, NH).

### EXAMPLE 2 (2-chloro-9-cyclopentyl-9H-purine-6-yl)-(6-furan-2-yl-pyridin-3-ylmethyl)-amine

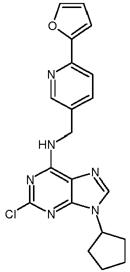

To the suspension of 9-cyclopentyl-2,6-dichloro-9*H*-purine (4.70 mmol) in a mixture of n-propanol (15 ml) and *N*,*N*-diisopropyl-N-ethylamine (9.40 mmol) [6-(2-furyl)pyrid-3-yl]methamine (5.17 mmol) was added. The suspension was heated with stirring in a sealed tube under an argon atmosphere at the temperature 120°C for 3 hours. After cooling to room temperature the reaction mixture was evaporated under reduced pressure and the residue was partitioned between water (50 ml) and dichloromethane (50 ml). The water phase was additionally extracted twice with dichloromethane. The combined organic phases were washed with water and brine and concentrated. Yield: 96%, m.p.: 119-122 °C. Elemental analysis: Calcd.for C₂₀H₁₉CIN₆O (394.86): C, 60.84; H, 4.85; N, 21.28. Found: C, 60.56; H, 4.92; N, 21.48. HPLC-MS (ESI+): 396 (97.6%). ¹H NMR (CDCl₃): 1.76-1.91(m, 6H), 2.22-2.28(m, 2H), 4.85-4.92(m, 3H, CH, CH₂), 6.54(d, J=3.42, 1H, ArH), 6.59(s(br), 1H, NH), 7.05(d, J=3.42, 1H, ArH), 7.53(d, J=3.42, 1H, ArH), 7.64-7.69(m, 2H, ArH), 7.75(d, J=6.27, 1H, ArH) 8.61(s, 1H, CH)

### EXAMPLE 3 (2-chloro-9-cyclopentyl-9H-purine-6-yl)-(6-thiophen-2-yl-pyridin-3-ylmethyl)-amine

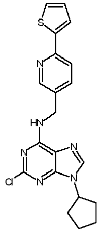

The 9-cyclopentyl-2,6-dichloro-9*H*-purine (1.48 mmol) was disslolved in a mixture of n-propanol (15.0 ml) and *N*,*N*-diisopropyl-*N*-ethylamine (6.0 mmol) and to the solution (6-thiophen-2-yl)pyrid-3-ylmethylamine dihydrochloride (1.63 mmol) was added. The reaction mixture was heated in a sealed tube under an argon atmosphere at 80°C for 16 hours. After cooling to room temperature was the reaction mixture diluted with water (30 ml) and the suspension was extracted twice with dichloromethane (25 ml). Combined organic phases were washed with water, brine, dried over sodium sulfate and evaporated under reduced pressure. The residue was used for further reactions without purification. Yield: 92%, m.p.: 111-114 °C. Elemental analysis: Calcd.for C₂₀H₁₉ClSN₆ (410.92): C, 58.46; H, 4.66; N, 20.45; S, 7.80. Found: C, 58.56; H, 4.72; N, 20.37, S, 7.55. HPLC-MS (ESI+): 411.3 (97.3%). ¹H NMR (DMSO-d₆):1.61-1.70(m, 2H), 1.78-1.96(m, 4H), 2.09-2.16(m, 2H), 4.64(d, J=5.37, 2H, CH₂), 4.77(qui, J=7.20, 1H, CH), 7.14(t, J=4.52, 1H, ArH), 7.59(d, J=5.01, 1H, ArH), 7.74(d, J=5.01, 1H, ArH), 7.80(d, J=4.52, 1H, ArH), 7.85(d, J=4.52, 1H, ArH), 8.27(s, 1H, CH), 8.51(s, 1H, ArH), 8.87(t, J=5.37, 1H, NH)

### EXAMPLE 4 N²-(4-amino-cyclohexyl)-N⁶-(6-bromo-pyridin-3-ylmethyl)-9-cyclopentyl-9H-purine-2,6-diamine

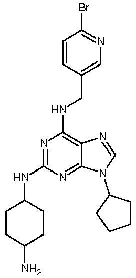

Well powdered (6-bromo-pyridin-3-ylmethyl)-(2-chloro-9-cyclopentyl-9*H*-purine-6-yl)-amine (7.36 mmol) and *trans*-1,4-diaminocyclohexane (110.0 mmol) were mixed and heated in a sealed tube under an argon atmosphere at 160°C for 4 hours. After cooling to 100 °C water (50 ml) was added to the reaction mixture and resulting suspension was extracted three times with ethyl acetate (50 ml). Combined organic phases were washed with water, brine, dried over sodium sulfate and evaporated under reduced pressure. The residue was dissolved in ethyl acetate (10 ml) and triturated with diethyl ether to obtain white crystalline mass which was filterd off and dried at 80°C for 4 hours. Yield: 33 %, m.p.: 114-116°C. Elemental analysis: Calcd.for C₂₂H₂₉BrN₈ (485.42): C, 54.43; H, 6.02; N, 23.08. Found: C, 54.29; H, 6.15; N, 23.00. HPLC-MS (ESI+): 487.3 (98.1%). ¹H NMR (CDCl₃): 1.13-1.29(m, 4H), 1.50(s(br), 2H, NH₂), 1.71-2.22(m, 12H), 2.75(sep, J=7.43, 1H, CH), 3.67(sex, J=7.52, 1H, CH), 4.59(d, J=7.52, 1H, NH), 4.70(qui, J=7.20, 1H, CH), 4.82(d, J=7.20, 2H, CH₂), 6.21(t, J=5.25, 1H, NH), 7.40(d, J=8.16, 1H, ArH), 7.55(dd, J=8.16, J'=2.4, 1H, ArH), 8.34(s, 1H, NH), 8.39(s, 1H, CH)

### EXAMPLE 5 4-{9-cyclopentyl-6-[(6-furan-2-yl-pyridin-3-ylmethyl)-amino]-9H-purine-2-ylamino}-cyclohexanol

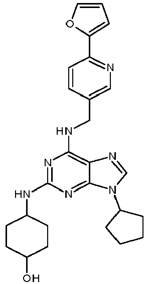

The *trans*-4-aminocyclohexan-1-ol hydrochloride (9.43 mmol) was suspended in methanol (10 ml) and to the suspension sodium methoxide (9.43 mmol) was added. The reaction mixture was stirred for 10 minutes at room temperature and sodium chloride was filtered off. The filtrate was evaporated under reduced pressure and to the residue (2-chloro-9-cyclopentyl-9*H*-purine-6-yl)-(6-furan-2-yl-pyridin-3-ylmethyl)-amine (0.25 mmol) and N-methylpyrrolidone (1 ml) was added. The reaction mixture was heated at 160 °C for 16 hours under an argon atmosphere. After cooling to room temperature water (10 ml) was added and resulting suspension was extracted twice with ethyl acetate (25 ml). Combined organic phases were washed with water, brine, dried over anhydrous sodium sulfate and evaporated under reduced pressure. The crude product was purified by column chromatography on silica, mobile phase chloroform-methanol (9:1). Yield: 33 %, m.p.: 164-166°C. Elemental analysis: Calcd.for C₂₆H₃₁N₇O₂ (473.57): C, 65.94; H, 6.60; N, 20.70. Found: C, 66.08; H, 6.48; N, 20.34. HPLC-MS (ESI+): 474.4 (99.6%). ¹H NMR (CDCl₃): 1.22(q, J= 10.2, 2H), 1.43(q, J=10.2, 2H), 1.72-1.81(m, 2H), 1.90-2.02(m, 6H), 2.11-2.25(m, 4H), 2.86(s(br), 1H, OH), 3.61-3.76(m, 2H), 4.64(d, J=7.68, 1H, NH), 4.69(qui, J=7.14, 1H, CH), 4.79(d, J=5.43, 2H, CH₂), 6.12(t, J=5.43, 1H, NH), 6.52(dd, J=3.39, J'=1.77, 1H, ArH), 7.02(d, J=3.39, 1H, ArH), 7.48(s, 1H, ArH), 7.52(d, J=3.39, 1H, ArH), 7.63(d, J=8.13, 1H, ArH), 7.73(dd, J=8.13, J'=2.07, 1H, ArH), 8.61(s, 1H, CH)

### EXAMPLE 6 Preparation of 1-{9-cyclopentyl-6-[(6-furan-2-yl-pyridin-3-ylmethyl)-amino]-9H-purine-2-ylamino}-2-methyl-propan-2-ol

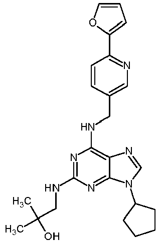

The mixture of (2-chloro-9-cyclopentyl-9*H*-purine-6-yl)-(6-furan-2-yl-pyridin-3-ylmethyl)-amine (2.53 mmol), 1-amino-2-methylpropan-2-ol (12.66 mmol) and *N,N-*diisopropyl-N-ethylamine (15.0 mmol) was heated at 160°C in a sealed tube under an argon atmosphere for 16 hours. After cooling to room temperature the reaction mixture was dissolved in a mixture of ethyl acetate (50 ml) and methanol (10 ml) and resulting solution was washed with water (50 ml). The water phase was then extracted twice with ethyl acetate (40 ml). Combined organic phases were washed with water, brine, dried over anhydrous sodium sulfate and eavporated under reduced pressure. The crude product was purified by column chromatography on silica using mobile phase chloroform - methanol (19:1, v/v). Yield: 37 %, m.p.: 128-129°C. Elemental analysis: Calcd.for C₂₄H₂₉N₇O₂ (447.53): C, 64.41; H, 6.53; N, 21.91. Found: C, 64.65; H, 6.44; N, 21.58. HPLC-MS (ESI+): 448.4 (99.5%). ¹H NMR (CDCl₃): 1.27(s, 6H, CH₃), 1.70-1.91(m, 6H), 2.20-2.35(m, 2H), 3.40(d, J=6.21, 2H, CH₂), 4.69(qui, J=6.42, 1H, CH), 4.79(s(br), 2H, CH₂), 5.24(t, J=6.21, 1H, NH), 5.61(s(br), 1H, OH), 6.04(s(br), 1H, NH), 6.54(t, J=3.42, 1H, ArH), 7.03(d, J=3.42, 1H, ArH), 7.50-7.54(m, 2H, ArH), 7.64(d, J=8.25, 1H, ArH), 7.74(dd, J=8.25, J'=3.42, 1H, ArH), 8.63(s, 1H, CH).

### EXAMPLE 7 Preparation of N²-(4-amino-cyclohexyl)-N⁶-{6-(2-amino-phenyl)-pyridin-3-ylmethyl]-9-cyclopentyl-9H-purine-2,6-diamine

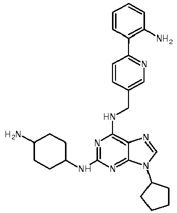

The *N*²-(4-amino-cyclohexyl)-*N*⁶-(6-bromo-pyridin-3-ylmethyl)-9-cyclopentyl-9*H-*purine-2,6-diamine (0.25 mmol), 2-aminophenylboronic acid hydrochloride (0.75 mmol), triphenylphosphine (0.50 mmol) and sodium carbonate (1.75 mmol) were suspended in a mixture of 1,2-dimethoxyethane (3.0 ml) and water (2.0 ml) and to this suspension bis(dibenzylideneacetone)palladium (7.50 µmol) was added under an argon atmosphere. The reaction mixture was heated in a sealed tube under argon atmosphere at 120°C for 18 hours. After cooling to room temperature the reaction mixture was diluted with water (25 ml) and resulting suspension was extracted twice with ethyl acetate (25 ml). Combined organic phases were washed with brine, dried over anhydrous sodium sulfate and evaporated under reduced pressure. The crude product was purified by column chromatography on silica, mobile phase chloroform - methanol - conc.ammonium hydroxide (8:2:0.05). Yield: 56%, m.p.: 173-175°C. Elemental analysis: Calcd.for C₂₈H₃₅N₉ (497.64): C, 67.58.; H, 7.09; N, 25.33. Found: C, 67.69; H, 7.19; N, 25.02. HPLC-MS (ESI+): 498.4 (99.9%). ¹H NMR (CDCl₃): 1.14-1.34(m, 4H), 1.71-2.05(m, 12H), 2.10-2.23(m, 4H, 2xNH₂), 2.75(sep, J=7.32, 1H, CH), 3.73(sex, J=7.52, 1H, CH), 4.59(d, J=7.52, 1H, NH), 4.70(qui, J=7.20, 1H, CH), 4.82(d, J=7.20, 2H, CH₂), 5.92(t, J=7.20, 1H, NH), 6.75-6.81(m, 2H, ArH), 7.20(t, J=7.89, 1H, ArH), 7.47-.751(m, 2H, ArH), 7.61(d, J=8.34, 1H, ArH), 7.79(d, J=8.34, 1H, ArH), 8.63(s, 1H, CH)

### EXAMPLE 8 Preparation of N²-(4-amino-cyclohexyl)-9-cyclopentyl-N⁶-(6-thiophen-2-yl-pyridin-3-ylmethyl)-9H-purine-2,6-diamine

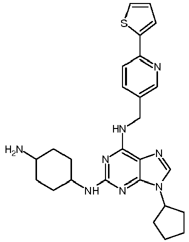

The mixture of well powdered (2-chloro-9-cyclopentyl-9*H*-purine-6-yl)-(6-thiophen-2-yl-pyridin-3-ylmethyl)-amine (0.75 mmol) and *trans*-1,4-diaminocyclohexane (10.95 mmol) was heated in a sealed tube under an argon atmosphere at 160°C for 3 hours. Aftre cooling to room temperature the reaction mixture was diluted with water (50 ml) and resulting suspension was extracted twice with ethyl acetate (50 ml). Combined organic phases were washed with water, brine, dried over anhydrous sodium sulfate and evaporated under reduced pressure. The crude product was purified by column chromatography on silica, mobile phase chloroform - methanol - conc.ammonium hydroxide (9:1:0.05). Yield: 88%, m.p.: 151-153 °C. Elemental analysis: Calcd.for C₂₆H₃₄N₈S (497.64): C, 63.64.; H, 6.98; N, 22.84; S, 6.53. Found: C, 63.72; H, 7.08; N, 23.02; S, 6.28. HPLC-MS (ESI+): 489.4 (99.9%). ¹H NMR (DMSO-d₆): 1.04-1.17(m, 4H), 1.64-2.05(m, 12H), 3.25-3.38(m, 3H, CH, NH₂), 3.54(sex, J=7.83, 1H, CH), 4.59-4.65(m, 3H, CH₂, CH), 6.09(d, J=7.83, 1H, NH), 7.13(t, J=4.05, 1H, ArH), 7.58(d, J=4.05, 1H, ArH), 7.71-.7.84(m, 4H, ArH), 7.90(s(br), 1H, NH), 8.51(s, 1H, CH).

### EXAMPLE 9 Preparation of N²-(4-amino-cyclohexyl)-9-cyclopentyl-N'-(6-furan-2-yl-pyridin-3-ylmethyl)-9H-purine-2, 6-diamine

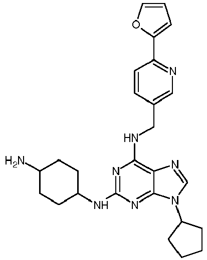

### Method A

The mixture of well powdered (2-chloro-9-cyclopentyl-9*H*-purine-6-yl)-(6-furan-2-yl-pyridin-3-ylmethyl)-amine (1.27 mmol) and *trans*-1,4-diaminocyclohexane (19.05 mmol) was heated in a sealed tube under an argon atmosphere at 160°C for 4 hours. After cooling to room temperature the reaction mixture was diluted with water (50 ml) and resulting suspension was extracted twice with ethyl acetate (50 ml). Combined organic phases were washed with water, brine, dried over anhydrous sodium sulfate and evaporated under reduced pressure. The crude product was purified by column chromatography on silica, mobile phase chloroform - methanol - conc.ammonium hydroxide (9:1:0.05). Yield: 89%, m.p.: 184-186°C. Elemental analysis: Calcd.for C₂₆H₃₂N₈O (472.59): C, 66.08.; H, 6.83; N, 23.71. Found: C, 66.32; H, 6.59; N, 23.99. HPLC-MS (ESI+): 473.5 (98.6%)

### Method B

The *N*²-(4-amino-cyclohexyl)-*N*⁶-(6-bromo-pyridin-3-ylmethyl)-9-cyclopentyl-9*H-*purine-2,6-diamine (0.41 mmol), 2-furanylboronic acid (1.24 mmol), triphenylphosphine (0.25 mmol) and sodium carbonate (1.70 mmol) were suspended in a mixture of 1,2-dimethoxyethane (3.0 ml) and water (2.0 ml) and to this ssuspension bis(dibenzylideneacetone)palladium (12.0 µmol) was added under an argon atmosphere. The reaction mixture was heated in a sealed tube under argon atmosphere at 120°C for 6 hours. After cooling to room temperature the reaction mixture was diluted with water (40 ml) and resulting suspension was extracted twice with ethyl acetate (50 ml). Combined organic phases were washed with brine, dried over anhydrous sodium sulfate and evaporated under reduced pressure. Crude product was purified by column chromatography on silica, mobile phase chloroform - methanol - conc.ammonium hydroxide (9:1:0.05). Yield: 78%, m.p.: 184-186°C. Elemental analysis: Calcd.for C₂₆H₃₂N₈O (472.59): C, 66.08.; H, 6.83; N, 23.71. Found: C, 66.25; H, 7.03; N, 23.54. HPLC-MS (ESI+): 473.5 (99.3%).

### Method C

To the suspension of *N*²-(4-amino-cyclohexyl)-*N*⁶-(6-bromo-pyridin-3-ylmethyl)-9-cyclopentyl-9H-purine-2,6-diamine (0.21 mmol), 2-furanylboronic acid (0.31 mmol), potassium phosphate trihydrate (0.80 mmol) and tetrabutylammonium bromide (0.003 mmol) in *N*,*N*-dimethylformamide (5.0 ml) palladium diacetate (2.5 µmol) was added under an argon atmosphere. The suspension was heated with stirring in a sealed tube at 120°C for 4 hours under an argon atmosphere. After cooling to room temperature the reaction mixture was diluted with water (20 ml) and the resulting suspension was extracted twice with ethyl acetate (25 ml). Combined organic phases were washed with brine, dried over anhydrous sodium sulfate and evaporated under redced pressure. The crude product was purified by column chromatography on silica, mobile phase chloroform - methanol - conc.ammonium hydroxide (9:1:0.05). Yield: 81%, m.p.: 180-183°C. Elemental analysis: Calcd.for C₂₆H₃₂N₈O (472.59): C, 66.08.; H, 6.83; N, 23.71. Found: C, 66.18; H, 6.59; N, 23.88. HPLC-MS (ESI+): 473.5 (99.8%). ¹H NMR (CDCl₃): 1.12-1.28(m, 4H), 1.71-2.15(m, 12H), 2.60-2.68(m, 3H, CH, NH₂), 3.68(sex, J=10.02, 1H, CH), 4.65-4.73(m, 4H, CH, CH₂, NH), 6.50(t, J=3.42, 1H, ArH), 6.62(s(br), 1H, NH), 7.00(s, 1H, ArH), 7.41-7.69(m, 4H, ArH), 8.57(s, 1H, CH).

### EXAMPLE 10 Preparation of N²-(4-amino-cyclohexyl)-9-cyclopentyl-N⁶-[6-(2-methoxy-phenyl)-pyridin-3-ylmethyl]-9H-purine-2,6-diamine

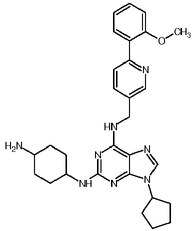

The *N*²-(4-amino-cyclohexyl)-*N*⁶-(6-bromo-pyridin-3-ylmethyl)-9-cyclopentyl-9*H-*purine-2,6-diamine (0.41 mmol), 2-methoxyphenylboronic acid (1.24 mmol), triphenylphosphine (0.25 mmol) and sodium carbonate (1.70 mmol) were suspended in a mixture of 1,2-dimethoxyethane (3.0 ml) and water (2.0 ml) and to this suspension bis(dibenzylideneacetone)palladium (12.0 µmol) was added under an argon atmosphere. The reaction mixture was heated in a sealed tube under argon atmosphere at 120°C for 3 hours. After cooling to room temperature the reaction mixture was diluted with water (25 ml) and resulting suspension was extracted twice with ethyl acetate (25 ml). Combined organic phases were washed with brine, dried over anhydrous sodium sulfate and evaporated under reduced pressure. Crude product was purified by column chromatography on silica, mobile phase chloroform - methanol - conc.ammonium hydroxide (9:1:0.05). Yield: 85%, m.p.: 184-186°C
Elemental analysis: Calcd.for C₂₉H₃₆N₈O (512.65): C, 67.94.; H, 7.08; N, 21.86. Found: C, 67.78; H, 7.01; N, 21.59. HPLC-MS (ESI+): 513.5 (99.6%). ¹H NMR (CDCl₃): 1.14-1.34(m, 4H), 1.71-2.22(m, 14H), 2.72(sep, J=5.87, 1H, CH), 3.75(sex, J=6.25, 1H, CH), 3.85(s, 3H, CH₃), 4.61(d, J=5.87, 1H, NH), 4.69(qui, J=6.87, 1H, CH), 4.82(d, J=7.20, 2H, CH₂), 6.00(s(br), J=7.20, 1H, NH), 7.00(d, J=8.22, 1H, ArH), 7.09(t, J=7.32, 1H, ArH), 7.35(t, J=7.32, 1H, ArH), 7.49(s, 1H, ArH), 7.70-7.74(m, 3H, ArH), 8.72(s, 1H, CH).

### EXAMPLE 11 Preparation of N²-(4-amino-cyclohexyl)-9-cyclopentyl-N⁶-[6-(2-hydroxy-phenyl)-pyridin-3-ylmethyl]-9H-purine-2,6-diamine

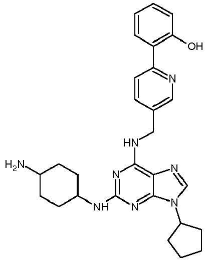

To the solution of *N*²-(4-amino-cyclohexyl)-9-cyclopentyl-*N⁶*-[6-(2-methoxy-phenyl)-pyridin-3-ylmethyl]-9*H*-purine-2,6-diamine (0.48 mmol) in dichloromethane (10 ml) boron tribromide (2.40 mmol) solution in dichloromethane (10 ml) was slowly added with stirring at room temperature. The mixture was stirred for further 18 hours and then methanol (20 ml) was added dropwise. The mixture was evaporated under reduced pressure and the residue was purificated by column chromatography on silica, mobile phase chloroform-methanol-ammonium hydroxide (4:1:0.025). Yield: 86 %, m.p.: 202-203°C. Elemental analysis: Calcd.for C₂₈H₃₄N₈O (498.62): C, 67.45.; H, 6.87; N, 22.47. Found: C, 67.28; H, 7.11 N, 22.41. HPLC-MS (ESI+): 499.5 (97.8%). ¹H NMR (CDCl₃): 1.16-1.40(m, 4H), 1.71-2.22(m, 12H), 2.49(s(br), 2H, NH₂), 2.80(sep, J=5.31, 1H, CH), 3.62(sex, J=7.25, 1H, CH), 4.61(d, J=7.77, 1H, NH), 4.69(qui, J=7.17, 1H, CH), 4.82(d, J=5.43, 2H, CH₂), 6.13(s(br), 1H, NH), 6.91(t, J=7.38, 1H, ArH), 7.02(d, J=8.19, 1H, ArH), 7.29(t, J=7.38, 1H, ArH), 7.50(s, 1H, ArH), 7.78(d, J=8.19, 1H, ArH), 7.82-7.86(m, 2H, ArH), 8.54(s, 1H, CH).

### EXAMPLE 12 Preparation of N²-(4-amino-cyclohexyl)-9-cyclopentyl-N⁶-[6-(3-fluorophenyl)-pyridin-3-ylmethyl]-9H-purine- 2,6-diamine

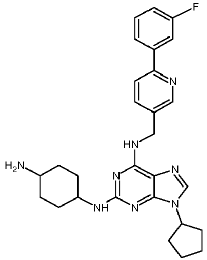

The *N*²-(4-amino-cyclohexyl)-*N*⁶-(6-bromo-pyridin-3-ylmethyl)-9-cyclopentyl-9*H-*purine-2,6-diamine (0.41 mmol), 3-flurophenylboronic acid (1.24 mmol), triphenylphosphine (0.25 mmol) and sodium carbonate (1.70 mmol) were suspended in a mixture of 1,2-dimethoxyethane (3.0 ml) and water (2.0 ml) and to this ssuspension bis(dibenzylideneacetone)palladium (12.0 µmol) was added under an argon atmosphere. The reaction mixture was heated in a sealed tube under argon atmosphere at 120°C for 18 hours. After cooling to room temperature the reaction mixture was diluted with water (25 ml) and resulting suspension was extracted twice with ethyl acetate (25 ml). Combined organic phases were washed with brine, dried over anhydrous sodium sulfate and evaporated under reduced pressure. The crude product was purified by column chromatography on silica, mobile phase chloroform - methanol - conc.ammonium hydroxide (9:1:0.05).Yield: 92%, m.p.: 121-122°C.
Elemental analysis: Calcd.for C₂₈H₃₃FN₈O (500.61): C, 67.18.; H, 6.64; N, 22.38. Found: C, 67.41; H, 6.69; N, 22.09. HPLC-MS (ESI+): 501.4 (99.5%). ¹H NMR (CDCl₃): 1.12-1.42(m, 4H), 1.71-2.21(m, 12H), 2.81(sex, J=5.87, 1H, CH), 3.12(s(br), 2H, NH₂), 3.73(sex, J=7.44, 1H, CH), 4.62-4.72(m, 2H, CH, NH), 4.81(d, J=5.77, 1H, CH₂), 6.33(t, J=5.77, 1H, NH), 7.12(t, J=8.25, 1H, ArH), 7.38-7.44(m, 2H, ArH), 7.61-7.77(m, 4H, ArH), 8.72(s, 1H, CH)

### EXAMPLE 13 Preparation of N²-(4-amino-cyclohexyl)-9-cyclopentyl-N⁶-(6-thiophen-3-yl-pyridin-3-ylmethyl)-9H-purine-2,6-diamine

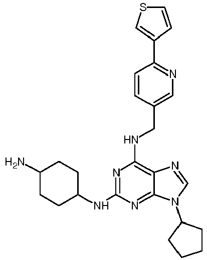

The *N*²-(4-amino-cyclohexyl)-*N*⁶-(6-bromo-pyridin-3-ylmethyl)-9-cyclopentyl-9*H-*purine-2,6-diamine (0.41 mmol), 3-thienylboronic acid (1.24 mmol), triphenylphosphine (0.25 mmol) and sodium carbonate (1.70 mmol) were suspended in a mixture of 1,2-dimethoxyethane (3.0 ml) and water (2.0 ml) and to this suspension bis(dibenzylideneacetone)palladium (12.0 µmol) was added under an argon atmosphere. The reaction mixture was heated in a sealed tube under an argon atmosphere at 120°C for 6 hours. After cooling to room temperature the reaction mixture was diluted with water (40 ml) and resulting suspension was extracted twice with ethyl acetate (50 ml). Combined organic phases were washed with brine, dried over anhydrous sodium sulfate and evaporated under reduced pressure. Crude product was purified by column chromatography on silica, mobile phase chloroform - methanol - conc.ammonium hydroxide (9:1:0.05). Yield: 68%, m.p.: 139-140 °C. Elemental analysis: Calcd.for C₂₆H₃₄N₈S (497.64): C, 63.64.; H, 6.98; N, 22.84; S, 6.53. Found: C, 63.62; H, 6.78; N, 22.59; S, 6.76. HPLC-MS (ESI+): 498.4 (98.9%). ¹H NMR (DMSO-d₆): 1.04-1.17(m, 4H), 1.64-2.05(m, 12H), 3.25-3.38(m, 3H, CH, NH₂), 3.54(sex, J=7.56, 1H, CH), 4.59-4.65(m, 3H, CH₂, CH), 6.09(d, J=7.56, 1H, NH), 7.11(s, J=4.12, 1H, ArH), 7.62(d, J=4.05, 1H, ArH), 7.72-.7.82(m, 4H, ArH), 7.90(s(br), 1H, NH), 8.53(s, 1H, CH).

### EXAMPLE 14 Preparation of N²-(4-amino-cyclohexyl)-9-cyclopentyl-N⁶-(6-furan-3-yl-pyridin-3-ylmethyl)-9H-purine-2, 6-diamine

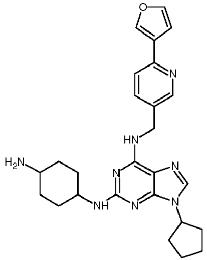

To the suspension of *N²*-(4-amino-cyclohexyl)-*N⁶*-(6-bromo-pyridin-3-ylmethyl)-9-cyclopentyl-9H-purine-2,6-diamine (0.21 mmol), 3-furanylboronic acid (0.31 mmol), potassium phosphate trihydrate (0.80 mmol) and tetrabutylammonium bromide (0.003 mmol) in *N*,*N*-dimethylformamide (5.0 ml) palladium diacetate (2.5 µmol) was added under an argon atmosphere. The suspension was heated with stirring in a sealed tube at 120°C for 4 hours under an argon atmosphere. After cooling to room temperature the reaction mixture was diluted with water (20 ml) and the resulting suspension was extracted twice with ethyl acetate (25 ml). Combined organic phases were washed with brine, dried over anhydrous sodium sulfate and evaporated under redced pressure. The crude product was purified by column chromatography on silica, mobile phase chloroform - methanol - conc.ammonium hydroxide (9:1:0.05). Yield: 56%, m.p.: 165-167°C. Calcd.for C₂₆H₃₂N₈O (472.59): C, 66.08.; H, 6.83; N, 23.71. Found: C, 66.01; H, 6.93; N, 23.51. HPLC-MS (ESI+): 473.26 (98.6%). ¹H NMR (CDCl₃): 1.12-1.28(m, 4H), 1.71-2.15(m, 12H), 2.60-2.68(m, 3H, CH, NH₂), 3.68(sex, J=10.00, 1H, CH), 4.65-4.73(m, 4H, CH, CH₂, NH), 6.50(t, J=3.42, 1H, ArH), 6.62(s(br), 1H, NH), 6.91(s, 1H, ArH), 7.00(s, 1H, ArH), 7.61-7.73(m, 4H, ArH), 8.57(s, 1H, CH).

**Table 1: Compounds Prepared by the Methods of Examples.**

| **No.** | **NAME OF COMPOUND** | **ELEMENTAL ANALYSES** | **MS (ZMD)** | |
|---|---|---|---|---|
| | | Calcd./Found [%] | [M-H]^{- a)} | [M+H]^{+ b)} |
| **BP8** | *N²*-(4-amino-cyclohexyl)-*N⁶*-[2,2']bipyridinyl-5-ylmethyl-9-cyclopentyl-9*H*-purine-2,6-diamine | C, 67.06/67.39; H, 6.88/6.95; N, 26.07/26.29 | 482.65 | 484.56 |
| **BP9** | *N²*-(4-amino-cyclohexyl)-9-cyclopentyl-*N⁶*-(6-pyrazol-1-yl-pyridin-3-ylmethyl)-9*H*-purine-2,6-diamine | C, 63.54/63.21; H, 6.82/6.55; N, 29.64/29.50 | 471.59 | 473.42 |
| **BP10** | 4-(5-{[2-(4-amino-cyclohexylamino)-9-cyclopentyl-9*H*-purine-6-ylamino]-methyl}-pyridin-2-yl)-benzoic acid | C, 66.14/66.15; H, 6.51/6.32; N, 21.28/21.03 | 525.58 | 527.78 |
| **BP14** | *N²*-(4-amino-cyclohexyl)-9-cyclopentyl-*N⁶*-(6-furan-2-yl-pyridin-3-ylmethyl)-9*H*-purine-2,6-diamine | C, 66.08/66.29; H, 6.83/6.74; N, 23.71/23.55 | 471.63 | 473.68 |
| **BP15** | *N²*-(2-amino-cyclohexyl)-9-cyclopentyl-*N⁶*-[6-(2-methoxy-phenyl)-pyridin-3-ylmethyl]-9*H*-purine-2,6-diamine | C, 67.94/67.62; H, 7.08/6.86; N, 21.86/21.41 | 511.65 | 513.78 |
| **BP18** | *N²*-(4-amino-cyclohexyl)-9-cyclopentyl-*N*⁶-[6-(2-methoxy-phenyl)-pyridin-3-ylmethyl]-9*H*-purine-2,6-diamine | C, 67.94/67.82; H, 7.08/7.36; N, 21.86/21.55 | 511.68 | 513.74 |
| **BP19** | *N*²-(4-amino-cyclohexyl)-9-cyclopentyl-*N*⁶-[6-(3-fluoro-phenyl)-pyridin-3-ylmethyl]-9*H*-purine-2,6-diamine | C, 67.18/66.94; H, 6.64/6.35; N, 22.38/22.65 | 499.69 | 501.65 |
| **BP20** | *N*²-(4-amino-cyclohexyl)-9-cyclopentyl-*N⁶*-[6-(2-hydroxy-phenyl)-pyridin-3-ylmethyl]-9*H*-purine-2,6-diamine | C, 67.45/67.21; H, 6.87/6.53; N, 22.47/22.41 | 497.62 | 499.58 |
| **BP21** | 4-{9-cyclopentyl-6-[(6-furan-2-yl-pyridin-3-ylmethyl)-amino]-9*H*-purine-2-ylamino}-cyklohexanol | C, 65.94/65.95; H, 6.60/6.47; N, 20.70/20.50 | 472.55 | 474.60 |
| **BP22** | *N²*-(4-amino-cyclohexyl)-9-cyclopentyl-*N⁶*-[6-(2-amino-phenyl)-pyridin-3-ylmethyl]-9*H*-purine-2,6-diamine | C, 67.58/67.96; H, 7.09/7.00; N, 25.33/25.12 | 496.50 | 498.55 |
| **BP25** | *N*²-(2-amino-cyclohexyl)-9-cyclopentyl-*N*⁶-[6-(2-hydroxy-phenyl)-pyridin-3-ylmethyl]-9*H*-purine-2,6-diamine | C, 67.45/67.33; H, 6.87/6.83; N, 22.47/22.39 | 497.60 | 499.54 |
| **BP27** | *N*²-(3-amino-cyclohexyl)-9-cyclopentyl-*N*⁶-[6-(2-methoxy-phenyl)-pyridin-3-ylmethyl]-9*H*-purine-2,6-diamine | C, 67.94/67.81; H, 7.08/7.09; N, 21.86/21.55 | 511.70 | 513.80 |
| **BP28** | *N*²-(3-amino-cyclohexyl)-9-cyclopentyl-*N⁶*-[6-(2-hydroxy-phenyl)-pyridin-3-ylmethyl]-9*H*-purine-2,6-diamine | C, 67.45/67.33; H, 6.87/6.83; N, 22.47/22.39 | 497.60 | 499.54 |
| **BP29** | (2-chlor-9-cyclopentyl-9*H*-purine-6-yl)-(6-thiophen-2-yl-pyridin-3-ylmethyl)-amine | C, 58.46/58.49; H, 4.66/4.39; N, 20.45/20.26 | 409.85 | 411.96 |
| **BP30** | *N*²-(4-amino-cyclohexyl)-9-cyclopentyl-*N⁶*-(6-thiophen-2-yl-pyridin-3-ylmethyl)-9*H*-purine-2,6-diamine | C, 63.91/63.72; H, 6.60/6.51; N, 22.93/22.71; S, 6.56/6.24 | 487.62 | 489.72 |
| **BP33** | 4-(9-cyclopentyl-6-{[6-(2-methoxy-phenyl)-pyridin-3-ylmethyl]-amino}-9*H*-purine-2-ylamino)-cyklohexanol | C, 67.81/67.49; H, 6.87/6.55; N, 19.09/19.27 | 512.57 | 514.80 |
| **BP34** | 4-(9-cyclopentyl-6-{[6-(2-hydroxy-phenyl)-pyridin-3-ylmethyl]-ammo}-9*H*-purine-2-ylamino)-cyklohexanol | C, 67.31/67.15; H, 6.66/6.47; N, 19.62/19.53 | 498.52 | 500.57 |
| **BP35** | *N*²-(4-amino-cyclohexyl)-9-cyclopentyl-*N*⁶-(6-thiophen-3-yl-pyridin-3-ylmethyl)-9*H*-purine-2,6-diamine | C, 63.91/64.12; H, 6.60/6.91; N, 22.93/22.68; S, 6.56/6.35 | 487.62 | 489.72 |
| **BP36** | *N*²-(4-amino-cyclohexyl)-9-cyclopentyl-*N⁶*-(6-furan-3-yl-pyridin-3-ylmethyl)-9*H*-purine-2,6-diamine | C, 66.08/65.92; H, 6.83/6.59; N, 23.71/23.41 | 471.55 | 473.65 |
| **BP37** | 2-{4-[9-Cyclopentyl-6-(4-furan-2-yl-benzylamino)-9*H*-purine-2-yl]-piperazin-1-yl}-ethanol | C, 66.51/66.55; H, 6.82/6.69; N, 20.11/20.01 | 486.60 | 488.57 |
| **BP43** | 1-(9-cyclopentyl-6-{[6-(3-fluoro-phenyl)-pyridin-3-ylmethyl]-amino}-9*H*-purine-2-ylamino)-2-methyl-propan-2-ol | C, 65.67/65.42; H, 6.36/6.16; N, 20.62/20.48 | 474.52 | 476.68 |
| **BP44** | 1-(9-cyclopentyl-6-{[6-(2-methoxy-phenyl)-pyridin-3-ylmethyl]-amino}-9*H*-purine-2-ylamino)-2-methyl-propan-2-ol | C, 66.51/66.37; H, 6.82/6.41; N, 20.11/19.94 | 486.59 | 488.62 |
| **BP45** | 1-(9-cyclopentyl-6-{[6-(2-hydroxy-phenyl)-pyridin-3-ylmethyl]-amino}-9*H*-purine-2-ylamino)-2-methyl-propan-2-ol | C, 65.94/65.68; H, 6.60/6.65; N, 20.70/20.54 | 472.55 | 474.60 |
| **BP47** | 4-{9-cyclopentyl-6-[(6-furan-2-yl-pyridin-3-ylmethyl)-amino]-9*H*-purine-2-ylamino}-2-methyl-butan-2-ol | C, 65.06/65.21; H, 6.77/6.51; N, 21.24/21.11 | 460.51 | 462.62 |
| **BP48** | 4-(9-cyclopentyl-6-{[6-(2-amino-phenyl)-pyridin-3-ylmethyl]-amino}-9*H*-purine-2-ylamino)-2-methyl-butan-2-ol | C, 66.64/66.58; H, 7.04/7.25; N, 23.03/22.86 | 485.59 | 487.63 |
| **BP49** | *N*²-(2-amino-propyl)-9-cyclopentyl-*N⁶*-(6-thiophen-2-yl-pyridin-3-ylmethyl)-9*H*-purine-2,6-diamine | C, 61.58/61.28; H, 6.29/6.05; N, 24.98/24.78 | 447.62 | 449.63 |
| **BP50** | *N*²-(2-amino-propyl)-9-cyclopentyl-*N*⁶-[6-(2-methoxy-phenyl)-pyridin-3-ylmethyl]-9*H*-purine-2,6-diamine | C, 66.08/66.29; H, 6.83/6.59; N, 23.71/23.56 | 471.55 | 473.63 |
| **BP51** | *N*²-(2-amino-propyl)-9-cyclopentyl-*N*⁶-[6-(2-hydroxy-phenyl)-pyridin-3-ylmethyl]-9*H*-purine-2,6-diamine | C, 65.48/65.49; H, 6.59/6.47; N, 24.44/24.11 | 457.42 | 459.45 |
| **BP57** | *N²*-(3-amino-propyl)-9-cyclopentyl-*N*⁶-(6-furan-2-yl-pyridin-3-ylmethyl)-9*H*-purine-2,6-diamine | C, 63.87/63.65; H, 6.53/6.54; N, 25.91/25.76 | 431.50 | 433.58 |
| **BP58** | *N*²-(3-amino-propyl)-9-cyclopentyl-*N*⁶-[6-(3-fluor-phenyl)-pyridin-3-ylmethyl]-9*H*-purine-2,6-diamine | C, 65.20/65.03; H, 6.35/6.08; N, 24.33/24.59 | | |
| **BP62** | *N*²-(2-amino-ethyl)-9-cyclopentyl-*N*⁶-(6-thiophen-2-yl-pyridin-3-ylmethyl)-9*H*-purine-2,6-diamine | C, 60.81/60.62; H, 6.03/5.89; N, 25.79/25.51; S, 7.38/7.02 | 433.55 | 435.57 |
| **BP63** | 4-(5-{[2-(2-amino-ethyl)-9-cyclopentyl-9H-purine-6-ylamino]-methyl}-pyridin-2-yl)-benzoic acid | C, 63.54/63.82; H, 5.97/6.12; N, 23.71/23.46 | 471.56 | 473.60 |
| **BP70** | 3-{9-cyclopentyl-6-[(6-thiophen-3-yl-pyridin-3-ylmethyl)-amino]-9*H*-purine-2-ylamino}-propan-1-ol | C, 61.45/61.59; H, 6.05/5.89; N, 21.81/21.54; S, 7.13/7.00 | 448.60 | 450.55 |
| **BP71** | 3-{9-cyclopentyl-6-[(6-furan-2-yl-pyridin-3-ylmethyl)-amino]-9*H*-purine-2-ylamino}-propan-1-ol | C, 63.72/63.98; H, 6.28/5.96; N, 22.62/22.45 | 432.51 | 434.55 |
| **BP72** | 3-{9-cyclopentyl-6-[(6-furan-3-yl-pyridin-3-ylmethyl)-amino]-9*H*-purine-2-ylamino}-propan-1-ol | C, 63.72/63.58; H, 6.28/6.53; N, 22.62/22.81 | 432.51 | 434.55 |
| **BP73** | 3-(9-cyclopentyl-6-{[6-(2-methoxy-phenyl)-pyridin-3-ylmethyl]-amino}-9*H*-purine-2-ylamino)-propan-1-ol | C, 65.94/65.74; H, 6.60/6.41; N, 20.70/20.46 | 472.57 | 474.58 |
| **BP74** | 3-(9-cyclopentyl-6-{[6-(2-hydroxy-phenyl)-pyridin-3-ylmethyl]-amino}-9*H*-purine-2-ylamino)-propan-1-ol | C, 65.34/65.02; H, 6.36/6.54; N, 21.34/21.08 | 458.56 | 460.58 |
| **BP81** | (*R*)-3-{9-cyclopentyl-6-[(6-thiophen-2-yl-pyridin-3-ylmethyl)-amino]-9*H*-purine-2-ylamino}-pentan-2-ol | C, 62.87/62.58; H, 6.54/6.55; N, 20.53/20.48; S, 6.71/6.57 | 476.60 | 478.64 |
| **BP82** | (*R*)-3-{9-cyclopentyl-6-[(6-thiophen-3-yl-pyridin-3-ylmethyl)-amino]-9*H*-purine-2-ylamino}-pentan-2-ol | C, 62.87/62.88; H, 6.54/6.32; N, 20.53/20.57; S, 6.71/6.74 | 476.60 | 478.64 |
| **BP83** | (*R*)-3-(9-cyclopentyl-6-{[6-(3-fluor-phenyl)-pyridin-3-ylmethyl]-amino}-9*H*-purine-2-ylamino)-pentan-2-ol | C, 66.24/66.58; H, 6.59/6.64; N, 20.03/19.86 | 488.63 | 490.65 |
| **BP84** | (*R*)-3-(9-cyclopentyl-6-{[6-(2-methoxy-phenyl)-pyridin-3-ylmethyl]-amino}-9*H*-purine-2-ylamino)-pentan-2-ol | C, 67.04/67.10; H, 7.03/7.25; N, 19.55/19.19 | 500.65 | 502.66 |
| **BP85** | (*R*)-3-(9-cyclopentyl-6-{[6-(2-hydroxy-phenyl)-pyridin-3-ylmethyl]-amino}-9*H*-purine-2-ylamino)-pentan-2-ol | C, 66.51/66.83; H, 6.82/6.94; N, 20.11/20.03 | 486.60 | 488.62 |
| **BP109** | (6-bromo-pyridin-3-ylmethyl)-(2-chloro-9-cyclopentyl-9*H*-purine-6-yl)-amine | C, 47.14/47.28; H, 3.96/3.67; N, 20.61/20.19 | 406.70 | 408.82 |
| **BP110** | *N*²-(4-amino-cyclohexyl)-*N⁶*-(6-bromo-pyridin-3-ylmethyl)-9-cyclopentyl-9*H*-purine-2,6-diamine | C, 54.43/54.62; H, 6.02/5.87; N, 23.08/23.19 | 484.42 | 486.39 |
| **BP116** | (2-chloro-9-cyclopentyl-9*H*-purine-6-yl)-(6-furan-2-yl-pyridin-3-ylmethyl)-amine | C, 60.84/60.61; H, 4.85/4.87; N, 21.28/21.11 | 393.85 | 395.92 |
| **BP117** | 1-{9-cyclopentyl-6-[(6-furan-2-yl-pyridin-3-ylmethyl)-amino]-9*H*-purine-2-ylamino}-2-methyl-propan-2-ol | C, 64.41/64.65; H, 6.53/6.44; N, 21.91/21.58 | 446.58 | 448.60 |
| **BP131** | (*R*)-2-(9-cyclopentyl-6-{[6-(3-fluoro-phenyl)-pyridin-3-ylmethyl]-amino}-9*H*-purine-2-ylamino)-butan-1-ol | C, 65.67/65.52; H, 6.36/6.12; N, 20.62/20.14 | 474.56 | 476.60 |
| **BP132** | (*R*)-2-(9-cyclopentyl-6-{[6-(2-methoxy-phenyl)-pyridin-3-ylmethyl]-amino}-9*H*-purine-2-ylamino)-butan-1-ol | C, 66.51/66.28; H, 6.82/6.49; N, 20.11/20.01 | 486.60 | 488.58 |
| **BP133** | (*R*)-2-(9-cyclopentyl-6-{[6-(2-hydroxy-phenyl)-pyridin-3-ylmethyl]-amino}-9*H*-purine-2-ylamino)-butan-1-ol | C, 65.94/65.81; H, 6.60/6.49; N, 20.70/20.51 | 472.57 | 474.59 |
| **BP153** | (S)-3-{9-Cyclopentyl-6-[(6-furan-2-yl-pyridin-3-ylmethyl)-amino]-9*H*-purine-2-ylamino}-2,4-dimethyl-pentan-2-ol | C, 66.23/66.01; H, 7.21/7.38; N, 20.02/19.83 | 488.61 | 490.53 |
| **BP154** | (S)-2-{9-Cyclopentyl-6-[(6-thiophen-2-yl-pyridin-3-ylmethyl)-amino]-9*H*-purine-2-ylamino}-3-methyl-butan-1-ol | C, 62.87/62.80; H, 6.54/6.39; N, 20.53/20.72 | 476.64 | 478.63 |

| | | | | |
|---|---|---|---|---|
| a) solution: MeOH p.a. + HCOOH; b) solution: MeOH p.a. + H₂O + NH₃ | | | | |

### EXAMPLE 15 In vitro Cytotoxic Activity of Novel Compounds

Cytotoxicity of the compounds is the major property determining their anticancer effect in vivo. One of the parameters used, as the basis for cytotoxicity assays, is the metabolic activity of viable cells. For example, a microtiter assay, which uses (3-(4,5-Dimethylthiazol-2-yl)-2,5-diphenyltetrazolium bromide (MTT), is widely used to quantitate cell proliferation and cytotoxicity. For instance, this assay is used in drug screening programs and in chemosensitivity testing. Because only metabolically active living cells reduce MTT to correspond purple formazan dye, these assays detect viable cells exclusively. The quantity of reduced MTT corresponds to the number of vital cells in the culture.

We have been using the following cell lines: HUH-7 and PLC/PRF/5 cell lines were maintained in DMEM supplemented with 10% fetal bovine serum, penicillin (100 U/ml) and streptomycin (100 µg/ml). Unique HCC-1.2 (3p) and HCC-1.1 (3sp) cell lines (Zilj et al., 2009, Future Oncol, 5(8):1169-79) and Hep3B cells were cultivated in RPMI supplemented with 10% fetal bovine serum, penicillin (100 U/ml) and streptomycin (100 µg/ml). HepG2 cell line was maintained in EMEM supplemented with 10% fetal bovine serum, sodium pyruvate (0.11 g/1), penicilin (100 U/ml) and streptomycin (100 µg/ml). All cell lines were cultivated at 37 °C in 5% CO₂. For cytotoxicity assays, 3000 cells (10000 cells in case of HepG2 cell line) were seeded into each well of 96 well plate and the next day tested compounds were added at various concentrations in triplicates. Three days after drug addition MTT stock solution (5 mg/ml) was added into each well and incubated for 4 h. After this incubation period, produced formazan was dissolved by DMSO and final absorbance was measured at 570 nm with Synergy H4 Hybrid Multi-Mode Microplate Reader. The EC₅₀ value, the drug concentration lethal to 50% of the tumour cells, was calculated from the obtained dose response curves.

Cytoxicity of novel compounds was tested on a panel of cell lines of different histogenetic origin. Significant activities were found in all hepatocarcinoma tumour cell lines tested (for example see Tab. 2). Notably, the higher effectiveness of novel derivatives was also found in cell lines bearing various mutations or deletions in cell cycle associated proteins, e.g. Hep3B, PLC-PRF-5, HepG2, HUH-7, AKH3p, AKH3sp (Puisieux et al., 1993, FASEB J. Nov;7(14):1407-13). It indicates that these substances should be equally effective in tumours with various statuses of tumour suppressor and cell cycle genes, namely p53, Rb, etc.

**Table 2: In vitro antiproliferative activity of selected novel substituted 2-substituted-6-biarylmethylamino-9-cyclopentyl-9H-purines in different hepatocellular carcinoma cell lines. (CR8 is [[9-(1-methylethyl)-6-[[[4-(2-pyridinyl)phenyl]methyl]amino]-9H-purin-2-yl] amino]-1-butanol)**

| Compound | Inhibitory concentration IC₅₀ (nM) | | | | | |
|---|---|---|---|---|---|---|
| | **Hep3B** | **PLC/PRF/5** | **HepG2** | **HUH-7** | **3p** | **3sp** |
| roscovitine | >10000 | >10000 | n.a. | >10000 | >10000 | >10000 |
| CR8 | >2000 | | | | | |
| BP14 | 58 | 7 | 211 | 373 | 410 | 513 |
| BP18 | 233 | 22 | 417 | 618 | 791 | 791 |
| BP19 | 85 | 9 | 111 | 281 | 191 | 410 |
| BP20 | 32 | 7 | 68 | 182 | 132 | 197 |
| BP21 | 487 | 46 | 38 | 155 | 167 | 408 |
| BP22 | 487 | 280 | >1000 | 642 | 436 | 516 |
| BP29 | 792 | n.a. | n.a. | n.a. | n.a. | 843 |
| BP30 | 77 | 15 | 206 | 184 | 205 | 476 |
| BP35 | 207 | 37 | 278 | 257 | 218 | 328 |
| BP36 | 130 | 18 | 131 | 332 | 258 | 424 |
| BP110 | 488 | 400 | >1000 | 513 | 334 | 515 |
| BP116 | 298 | n.a. | n.a. | n.a. | n.a. | 371 |

| | | | | | | |
|---|---|---|---|---|---|---|
| *n.a.: not analyzed* | | | | | | |

**Table 3: In vitro antiproliferative activity of reference molecules in different hepatocellular carcinoma cell lines**

| Reference molecule | Inhibitory concentration IC₅₀ (nM) | | | | | |
|---|---|---|---|---|---|---|
| | Hep3B | PLC/PRF/5 | HepG2 | HUH-7 | AKH3p | AKH3sp |
| doxorubicin | 0.46 | 0.67 | 1.38 | 1.25 | 0.54 | 0.27 |
| cisplatin | 1.28 | 10.15 | 1.38 | 3.62 | 2.71 | 0.72 |
| sorafenib | 3.74 | 4.06 | 5.85 | 2.86 | 4.42 | 6.39 |

### EXAMPLE 16 Kinase inhibitory activities of novel compounds

CDK2/Cyclin E kinase was produced in Sf9 insect cells via baculoviral infection and purified on a NiNTA column (Qiagen). CDK5/p35, CDK7Cyclin H/MAT1 and CDK9/Cyclin T1 was purchased from ProQinase GmbH. The kinase reactions were assayed with 1 mg/mL histone H1 (for CDK2 and CDK5) or (YSPTSPS)₂KK peptide (for CDK7 and CDK9) in the presence of 15/0.15/1.5/1.5 µM ATP (for CDK2/CDK5/CDK7CDK9), 0.05 µCi [γ-³³P]ATP and of the test compound in a final volume of 10 µL, all in a reaction buffer (60 mM HEPES-NaOH, pH 7.5, 3 mM MgCl₂, 3 mM MnCl₂, 3 µM Na-orthovanadate, 1.2 mM DTT, 2.5 µg / 50 µl PEG_{20.000}). The reactions were stopped by adding 5 µL of 3% aq H₃PO₄. Aliquots were spotted onto P-81 phosphocellulose (Whatman), washed 3x with 0.5% aq H₃PO₄ and finally air-dried. Kinase inhibition was quantified using digital image analyzer FLA-7000 (Fujifilm) and expressed as a residual activity of kinase or as IC₅₀, the concentration of the test compounds required to decrease the CDK by 50%.

As shown in Tab. 4 and 5, all compounds potently inhibited not only CDK1 and CDK2 in nanomolar ranges but also exhibited a strong activity towards others CDKs that are involved in other important biological processes. Inhibition of transcriptional CDK7/9 leads to downregulation of short half-life proteins connected to apoptosis (Mcl-1, XIAP) that has been shown to be critical for survival of cancer cells especially those causing multiple myeloma and chronic lymphocytic leukemia (Chen et al., Blood. 2005 Oct 1;106(7):2513-9; MacCallum et al., Cancer Res. 2005 Jun 15;65(12):5399-407; Manohar et al., Leuk Res. 2011 Jun;35(6):821-30). CDK5, so far known as a regulator of neuronal processes, plays also a key role in regulation of endothelial cell migration and tube formation, two essential steps of cellular angiogenesis (Liebl et al., J Biol Chem. 2010 Nov 12;285(46):35932-43). Therefore we investigated the inhibitory activity of the most potent compounds towards all CDKs and studied their effects to transcription and angiogenesis.

**Table 4: Kinase inhibitory activity of selected 2-substituted-6-biarylmethylamino-9-cyclopentyl-9H-purines expressed as IC₅₀.**

| Compound | Kinase inhibition IC₅₀ (nM) | |
|---|---|---|
| | CDK1 | CDK2 |
| Roscovitine | >1000 | 160 |
| CR8 | 787 | 51 |
| BP14 | 50.0 | 10.0 |
| BP18 | 118 | 34.0 |
| BP19 | 77.0 | 14.0 |
| BP20 | 47.0 | 7.1 |
| BP21 | 215 | 23.0 |
| BP22 | 100 | 10.0 |
| BP30 | 49.0 | 4.0 |
| BP35 | 169 | 18.0 |
| BP36 | 66.0 | 8.0 |

**Table 5: Kinase inhibitory activity of selected 2-substituted-6-biarylmethylamino-9-cyclopentyl-9H-purines expressed as a residual activity of kinases CDK1/7/9.**

| Compound | Residual kinase activity (%) | | | | | |
|---|---|---|---|---|---|---|
| | CDK5 | | CDK7 | | CDK9 | |
| | 1000 nM | 100 nM | 1000 nM | 100 nM | 1000 nM | 100 nM |
| BP14 | 24.82 | 37.79 | 23.95 | 54.83 | 0.73 | 22.31 |
| BP18 | 23.58 | 49.19 | 24.13 | 54.07 | 1.03 | 32.65 |
| BP19 | 25.62 | 37.73 | 24.98 | 55.05 | 0.83 | 26.55 |
| BP20 | 19.36 | 47.64 | 17.27 | 48.04 | 0.59 | 21.06 |
| BP21 | 23.37 | 88.22 | 48.47 | 72.03 | 7.74 | 48.99 |
| BP22 | 89.00 | 87.75 | 93.86 | 70.04 | 82.80 | 93.40 |
| BP30 | 4.31 | 26.02 | 16.50 | 47.68 | 1.78 | 19.59 |
| BP35 | 8.70 | 65.04 | 33.47 | 64.07 | 2.39 | 32.80 |
| BP36 | 2.95 | 31.88 | 17.34 | 49.06 | 1.68 | 15.39 |
| BP117 | 39.63 | 76.73 | 68.34 | 76.35 | 23.18 | 52.68 |

### EXAMPLE 17 One-step cellular caspase-3/7 activity assay of novel 2-substituted-6-biarylmethylamino-9-cyclopentyl-9H-purines

Measurements of proapoptotic properties of new compounds were based on quantification of enzymativ activities of caspases, concretely caspases-3/7. Activity of cellular caspase-3/7 was measured according to Carrasco et al., 2003, BioTechniques, 34(5): 1064-67. Briefly, Hep3B and PLC/PRF/5 cells were incubated in the densities of 10000 cells/well in a 96-well plate overnight. Next day, the compounds in appropriate concentrations were added and cells were incubated for the 24 hours. After incubation, 3x caspase-3/7 assay buffer (150 mM HEPES pH 7.4, 450 mM NaCl, 150 mM KC1, 30 mM MgC12, 1.2 mM EGTA, 1.5% Nonidet P40, 0.3% CHAPS, 30% sucrose, 30 mM DTT, 3 mM PMSF) with 150 µM Ac-DEVD-AMC as a substrate (Sigma-Aldrich) was added to the wells and plates were incubated at 37 °C at room temperature. The caspase-3/7 activity was measured after 6 hours using Fluoroskan Ascent microplate reader (Labsystems) at 346 nm/442 nm (excitation/emission).

A fluorimetry-based caspase-3/7 activity assay in Hep3B (Tab. 6) and PLC/PRF/5 cells (Table 7) treated with 2-substituted-6-biarylmethylamino-9-cyclopentyl-9*H*-purine derivatives revealed potent dose-dependent activation of the caspases in mid-nanomolar ranges.

**Table 6: Relative caspase-3/7 activity in Hep3B cells after treatment with novel compounds.**

| Compound | Relative caspase-3/7 activity in Hep3B cell line Concentration (nM) | | | | | |
|---|---|---|---|---|---|---|
| | 0 | 100 | 200 | 400 | 800 | 1600 |
| BP14 | 1.00 | 0.99 | 3.60 | 6.55 | 9.00 | 9.50 |
| BP18 | 1.00 | - | 0.89 | 3.19 | 6.05 | 8.59 |
| BP19 | 1.00 | 0.90 | 4.82 | 6.60 | 9.80 | 9.45 |
| BP20 | 1.00 | 4.42 | 5.37 | 8.25 | 9.63 | 9.95 |
| BP21 | 1.00 | - | 1.15 | 1.18 | 4.36 | 7.19 |
| BP22 | 1.00 | - | 0.81 | 0.80 | 0.81 | 1.18 |
| BP30 | 1.00 | 2.99 | 5.32 | 8.08 | 9.00 | 9.43 |
| BP35 | 1.00 | - | 1.01 | 5.59 | 6.07 | 9.10 |
| BP36 | 1.00 | 2.67 | 6.19 | 6.55 | 9.80 | 9.75 |
| BP117 | 1.00 | - | 0.77 | 0.89 | 1.06 | 2.00 |

**Table 7: Relative caspase-3/7 activity in PLC/PRF/5 cells after treatment with novel compounds.**

| Compound | Relative caspase-3/7 activity in PLC/PRF/5 cell line Concentration (nM) | | | | | | |
|---|---|---|---|---|---|---|---|
| | 0 | 40 | 80 | 160 | 320 | 640 | 1280 |
| BP14 | 1.00 | 1.99 | 2.86 | >5.00 | >5.00 | >5.00 | >5.00 |
| BP18 | 1.00 | 0.97 | 2.40 | >5.00 | >5.00 | >5.00 | >5.00 |
| BP19 | 1.00 | 1.78 | 2.39 | >5.00 | >5.00 | >5.00 | >5.00 |
| BP20 | 1.00 | 3.15 | 2.66 | >5.00 | >5.00 | >5.00 | >5.00 |
| BP21 | 1.00 | 0.69 | 1.20 | 2.19 | 3.39 | >5.00 | >5.00 |
| BP22 | 1.00 | - | - | 0.72 | 1.08 | 1.92 | 3.50 |
| BP30 | 1.00 | 2.46 | 3.93 | >5.00 | >5.00 | >5.00 | >5.00 |
| BP35 | 1.00 | 1.10 | 2.37 | 2.97 | >5.00 | >5.00 | >5.00 |
| BP36 | 1.00 | 2.34 | >5.00 | >5.00 | >5.00 | >5.00 | >5.00 |
| BP117 | 1.00 | - | - | 0.79 | 1.17 | 2.60 | 2.99 |

### EXAMPLE 18 Effect of 2-substituted-6-biarylmethylamino-9-cyclopentyl-9H-purine BP14 on cellular apoptosis

Measurements of proapoptotic properties of BP14 were based on quantification of enzymatic activities of caspases-3/7. For caspase assays, treated cells were harvested by centrifugations and homogenized in an extraction buffer (10 mM KC1, 5 mM Hepes, 1 mM EDTA, 1 mM EGTA, 0.2% CHAPS, inhibitors of proteases, pH 7.4) on ice for 20 min. The homogenates were clarified by centrifugation at 10,000 g for 20 min at 4 °C, then proteins were quantified by the Bradford method and diluted to the same concentration. Lysates were then incubated for 5 h with 100 µM Ac-DEVD-AMC as substrate (Sigma-Aldrich) in an assay buffer (25 mM PIPES, 2 mM EGTA, 2 mM MgCl₂, 5 mM DTT, pH 7.3). The fluorescence of the product was measured using a Fluoroskan Ascent microplate reader (Labsystems, Helsinki, Finland) at 346 nm/442 nm (ex/em).

Compound BP14 strongly induces the activity of caspase-3/7 in Hep3B carcinoma cells; after 24 h treatment a twenty-fold increase at concentration of 3xIC₅₀ was observed in assay compared with the untreated control. The effect of BP14 on activation of caspases was determined also in other hepatocellular carcinoma cell lines (see Fig. 2).

Effect of BP14 on cell apoptosis was complemented by immunoblot analysis of selected apoptotic proteins. For immunoblotting, cell were detached with rubber policeman and washed three times with ice-cold PBS and lysed in buffer (50 mM Tris, pH 7,4, 250 mM NaCl, 5 mM EDTA, 50 mM NaF, 1 mM Na₃VO₄, 1% Nonidet P40) containing mixture of protease and phosphatase inhibitors (Sigma-Aldrich, USA). 20 µg of total proteins were separated by SDS-polyacrylamide gel electrophoresis (SDS-PAGE) and transferred onto nitrocellulose membranes. Membranes were blocked in 5% milk and 0.1% Tween 20 in PBS and probed overnight with specific antibodies for PARP-1 (clone F-2; Santa Cruz Biotechnology, USA), Mcl-1 (S-19; Santa Cruz Biotechnology, USA) and β-actin (C-4, Santa Cruz Biotechnology, USA). All primary antibodies were diluted in PBS containing 5% powdered milk; 0.1% Tween 20. Peroxidase conjugated rabbit anti-mouse immunoglobulin or porcine anti-rabbit immunoglobulin antisera (DAKO, Denmark) were used as the secondary antibodies and visualised with ECL reagents (Amersham-Pharmacia, Little Chalfont, UK).

Monitoring of the cleavage of PARP-1, a nuclear target of caspase-3, confirmed the above results. An appearance of the caspase-3-cleaved PARP-1 fragment at 89 kDa after cell exposure to BP14 was associated with a diminution of its fulllength form (Fig. 1) and is markedly observed in treated Hep3B and AHK3p cells. On the other hand no effect of protein level of PARP-1 was observed after treatment of HUH-7 cells with BP14 at concentration up to 3xIC₅₀ (µM). The activation of apoptosis was evident also from determination of the level of anti-apoptotic protein Mcl-1 that showed a large dose-dependent decrease in Hep3B, PLC/PRF/5 and AKH3p cell lines.

### EXAMPLE 19 Induction of tumour supressor p53 in hepatocarcinoma cancer cells

Stronger anticancer activity of the compounds is enhanced by its positive impact on stability and activity of the tumour suppressor p53. To measure p53-dependent transcriptional activity, β-galactosidase activity was quantified in the melanoma cell line Arn8, which had been established using stable transfection of cells with a p53-responsive reporter construct pRGCΔfoslacZ (Frebung et al., Cancer Res., 52, 1992-6976). Briefly, after 24 h incubation with the inhibitors the Arn8 cells were permeabilized with 0.3% Triton X-100 for 15 min and then 4-methylumbelliferon-ß-D-galactopyranoside was added as a substrate to the final concentration of 80 µM. After 1 h the fluorescence of product 4-methylumbelliferon was measured at 355/460 nm (ex/em) with a Fluoroskan Ascent microplate reader (Labsystems). Data in Tab. 8 present that series of 2-substituted-6-biarylmethylamino-9-cyclopentyl-9*H*-purine derivatives show a dose-dependent activatory effect on p53-regulated transcription, with the maximum impact observed between 100 and 500 nM concentrations for most of prepared compounds (Tab. 8).

**Table 8: The effect of selected novel 2-substituted-6-biarylmethylamino-9-cyclopentyl-9H-purine derivatives on induction of p53 protein in Arn8 cells stable transfected with a β-galactosidase reporter gene.**

| Compound | Concentration of maximum activation (nM) |
|---|---|
| roscovitine | >10000 |
| CR8 | 1600 |
| BP14 | 137 |
| BP18 | 510 |
| BP19 | 167 |
| BP20 | 150 |
| BP21 | 918 |
| BP22 | 373 |
| BP30 | 120 |
| BP35 | 230 |
| BP36 | 125 |
| BP117 | 950 |

### EXAMPLE 20 Novel 2-substituted-6-biarylmethylamino-9-cyclopentyl-9H-purine derivatives inhibit cellular transcription by reduction of phosphorylation of RNA polymerase II

We monitored levels of phosphorylation of RNA polymerase II, which is a substrate of CDK7 and CDK9, in cells treated with compound BP14 (Fig. 3). Immunoblotting analysis was performed as described in Example 33 with using appropriate antibodies for anti-phospho RNA polymerase II (S5) (Bethyl Laboratories, USA), anti-phospho RNA polymerase II (S2) (Bethyl Laboratories, USA), anti-RNA polymerase II (clone ARNA-3, Millipore) and β-actin (clone C4, Santa Cruz Biotechnology, USA).

Immunoblotting analysis revealed a rapid decrease in phosphorylation at serines 2 and 5 of RNA polymerase II mainly in AKH3sp and Hep3B cell lines (Figure 3), confirming cellular inhibition of these two kinases. Significant decrease in phophorylation of both forms of RNA polymerase II was observed in cells treated by BP14 in a concentration corresponding to IC₅₀ (24 h). Observed inhibition of cellular transcription was confirmed by detection of downregulation of Mcl-1 that belong to the group of anti-apoptotic proteins with short-half live (Fig. 1).

### EXAMPLE 21 Anti-angiogenic effect of novel derivatives

Novel 2-substituted-6-biarylmethylamino-9-cyclopentyl-9*H*-purine derivatives were tested for their potential anti-angiogenic properties; we analyzed its influence on the proliferation (Tab. 9), migration and tube formation of human umbilical vein endothelial cells (HUVEC). Confluent HUVECs were seeded in 96-well microtiter plates to detect their proliferation for 24 or 72 h using Calcein AM solution (Invitrogen) and a Fluoroskan Ascent microplate reader (Labsystems) as decribed previously (Krystof et al., 2011, Eur J Med Chem. 2011 Sep;46(9):4289-94).

**Table 9: In vitro antiproliferative activity of selected novel 2-substituted-6-biarylmethylamino-9-cyclopentyl-9H-purines in human umbilical vein endothelial cells (HUVECs).**

| **Compound** | **Inhibitory concentration for HUVEC cells - IC₅₀ (µM)*^{a}*** | |
|---|---|---|
| | **24h** | **72h** |
| doxorubicin | >6.6 | >6.6 |
| sorafenib | >50 | >10 |
| CR8 | >50 | 0.61 |
| BP14 | >50 | 0.05 |
| BP18 | >50 | 0.52 |
| BP19 | 21.8 | 0.37 |
| BP20 | 29.2 | 0.57 |
| BP30 | 20.4 | 0.08 |
| BP35 | >50 | 0.18 |
| BP36 | 20.8 | 0.19 |

| | | |
|---|---|---|
| *^{a}* Average values from three determinations | | |

### EXAMPLE 22 Anti-angiogenic effect of novel derivatives - migration scratch assay

Migration assay was performed as described previously (Krystof et al., 2011, Eur J Med Chem.;46(9):4289-94). Briefly, confluent HUVECs were scratched and immediately treated for 24 h with with various doses of compounds. After incubation each well was photographed using a DP Controller system (Olympus) connected to a Olympus BX50 microscope . Migration was expressed as the proportion of pixels of the wound area in the image that were not covered by cells using "*in house*" software.

The migration of VEGFstimulated HUVECs across a scratched area was inhibited by novel substituted 2-substituted-6-biarylmethylamino-9-cyclopentyl-9*H*-purines in a concentration of 100 and 1000 nM (Tab. 10). Significant inhibition of migration was primarily observed in cells treated by BP20 and BP30 at concentration 100 nM after 24 h treatment that did not affect cell viability (see Tab. 9).

**Table 10. Effect of selected novel substituted 2-substituted-6-biarylmethylamino-9-cyclopentyl-9H-purines on migration of human umbilical vein endothelial cells (HUVECs).**

| **Compound** | **Wound area*^{a}*** | |
|---|---|---|
| | **concentration (nM)** | |
| | **100** | **1000** |
| olomoucine | 0 | 0 |
| roscovitine | 0 | 0 |
| CR8 | 0 | + |
| BP14 | + | ++ |
| BP18 | + | ++ |
| BP19 | + | ++ |
| BP20 | ++ | ++ |
| BP30 | ++ | ++ |
| BP35 | 0 | ++ |
| BP36 | + | ++ |

| | | |
|---|---|---|
| *^{a}*++ open; + partly open, 0 closed | | |

### EXAMPLE 23 Anti-angiogenic effect of novel derivatives - tube formation assay

For an evaluation of tube formation, HUVEC cells in endothelial cell growth medium (ECGM) containing tested compound were seeded onto Matrigel® (BD) coated ibidi angiogenesis slides (15-well, ibidi GmbH, Munich, Germany). After 24 h, images were taken using the Olympus BX50 miscoscope with DP Controller system. Evaluation of formation of tubes was expressed as a number of tubes and number of nodes of treated cells compared with untreated control using specific "in *house*" software.

Many 2-substituted-6-biarylmethylamino-9-cyclopentyl-9*H*-purines significantly reduced HUVEC migration in a concentration of 10 nM or 100 nM (Fig. 5). As an example - several branching points, number of tubes, total tube length and mean tube length were significantly reduced by 24 h treatment with compounds BP14 and BP20 (Fig. 5).

### EXAMPLE 24 Anti-inflammatory activity of novel derivatives - reduction of E-selectin expression

For an evaluation of anti-inflammatory activity of 2-substituted-6-biarylmethylamino-9-cyclopentyl-9*H*-purines an expression of E-selectin (ELAM-1 - endothelial-leukocyte adhesion molecule 1) was analysed. ELAM-1 belong to the group of cell-surface glycoproteins that is rapidly induced by inflammatory cytokines e.g. tumor necrosis factor (TNF) during chronic or acute inflammation processes (Ley, Trends Mol Med. 2003 Jun;9(6):263-8). Therefore we evaluated an influence of novel compounds on expression of membrane-bound E-selectin on HUVECs after stimulation by TNF by highly sensitive ELISA-based assay.

Briefly, 96-well plate was coated with gelatine by applying 200 µl of 1.0% gelatine for 10 minutes at room temperature. 1 × 10⁴ HUVECs were seeded in each of the other wells in 200 µl medium and grown for 48 h to optimal confluence. Increasing concentrations of tested inhibitors were then added to the HUVEC-containing wells in triplicates, and the cells were incubated for 30 min, after which 10 ng/ml TNFα was added per well to stimulate NF-κB, and thus ELAM-1. After further 4 h incubation, the levels of ELAM-1 in each of the HUVEC-containing wells were determined by enzyme-linked activity assays (ELISAs). Cells were washed once with PBS and fixed with 0.1% glutaraldehyde (Sigma-Aldrich, Munich, Germany), for 15 min at room temperature. Then, cells were washed 3 x with 200 µl per well PBS/0.05% Tween 20, blocked with 200 µl/well 5% BSA/PBS for 1 h, and washed again 3 x with 200 µl per well PBS/0.05% Tween 20. Then, anti-ELAM-1 antibody (clone BBA-1, R&D Systems, Minneapolis, MN, USA) diluted 1:5000 in 0.1% BSA/PBS (100µl per well) was added for 1 h at room temperature and washed thereafter 5 x with 200 µl per well PBS/0.05% Tween 20. Subsequently, goat anti mouse-HRP antibody (Sigma-Aldrich, Munich, Germany) diluted 1:10000 in 0.1% BSA/PBS (100µl per well) was applied and the cells were incubated for 1 h in the dark at room temperature and, after decanting, washed five times with 200 µl per well PBS/0.05 % Tween 20. The HRP-activity of the cells in each of the wells was estimated using Fast-OPD (o-phenylenediamine dihydrochloride) (Sigma-Aldrich, Munich, Germany) assay as described (Gridling et al, Int J Oncol. 2009, Apr;34(4):1117-28) and absorbance was measured at OD₄₉₂ₙₘ in a vertical spectrophotometer. All data were normalized to positive control (cells treated by TNF without inhibitor) that represents 100% of inflammation.

Selected 2-substituted-6-biarylmethylamino-9-cyclopentyl-9*H*-purines rapidly decrease an expression of ELAM-1 in nanomolar concentration ranges that did not affect cell viability (data not shown). Results clearly show that most of new compounds are significantly more potent than the reference compound doxorubicin (DOX) or sorafenib (SOR) and e.g. purines BP36 and BP30 are among the most active in the series of compounds examined, with inhibition values below 25% (for treatment of 1µM of compound).

### EXAMPLE 25 BP-14 in hepatoma cells in vitro and in vivo

### Cell culture

The human hepatoma cell lines and HepG2, PLC/PRF/5 (PLC), Hep3B and 3sp (formerly described as HCC-1.1) were cultivated in RPMI 1640 and 10% fetal calf serum (FCS) as described (31, 32). All cells were kept at 37 °C and 5% CO₂ and were routinely screened for the absence of mycoplasma.

### Primary human hepatocytes (PHHs)

Non-neoplastic tissue samples from liver resections were obtained from patients undergoing partial hepatectomy for metastatic liver tumors of colorectal cancer. Experimental procedures were performed according to the guidelines of the charitable state controlled foundation HTCR (Human Tissue and Cell Research, Regensburg, Germany), with the informed patient's consent approved by the local ethical committee of the University of Regensburg. PHHs were isolated using a modified two-step EGTA/collagenase perfusion procedure as described previously (33). Viability of isolated PHHs was determined by trypan blue exclusion and cells with a viability of more than 85% were used for further work. Cells were plated on collagen-coated plates (BD Biosciences, San Jose, USA) at a density of 1.2 x 10⁵ cells/cm². The medium consisted of DMEM with 10% FCS, 2 mM L-glutamine, 100 mg/ml streptomycin, 100 U/ml penicillin and supplements as follows: 125 mU/ml insulin, 7.3 ng/ml glucagon and 0.8 µg/ml hydrocortisone. Cells were incubated at 37 °C in a humidified incubator with 5% CO₂ and media were changed daily.

### Therapeutic agents

BP-14 (N2-(4-aminocyclohexyl)-9-cyclopentyl-N6-[[6-(2-furyl)-3-pyridyl]methyl]purine-2,6-diamine) were synthesized by procedures as described here. Compounds were dissolved in dimethylsulfoxide (DMSO). The stock solution of 100 mM was diluted in assay buffer or in medium to concentrations indicated in the text. The maximum concentration of DMSO in the assays never exceeded 0.1%.

### Determination of cell viability and inhibitory concentrati (IC)₅₀

Cell viability was determined using the 3-(4,5 dimethylthiazol-2-yl)-2,5-diphenyltetrazolium bromide (MTT) assay. Briefly, cells were seeded in triplicates at a density of 6 x 10³ cells per well. After 24 hours, cells were incubated with drug-containing medium for 72 hours. Cells were incubated with MTT solution (5 mg/ml; Sigma, St. Louis, USA) and medium was replaced with DMSO after five hours. The absorbance was measured at 620 nm by employing a microplate reader (Asys HiTech, Salzburg, Austria). MTT assays were repeated 3 times for each drug application and untreated cells were used as reference. IC₅₀ values were obtained by log-linear interpolation of data points and are depicted by dose-response curves using the software GraphPad Prism^{®} 5.01.

### Kinase inhibition assays using cell-free extracts

Whole cell extracts were prepared by lysing Hep3B cells with a buffer containing 20 mM Tris pH 8.0, 100 mM NaCl, 1 mM EDTA and 0.5% NP-40. 100 µg of extract was used for immunoprecipitation at 4 °C for 4 hours either with 1 µg of the anti-CDK2 antibody M2 (Santa Cruz Biotechnology, Santa Cruz, USA) or with 1 µg of the anti-cyclin B1 antibody GNS1 (Santa Cruz Biotechnology, Santa Cruz, USA). The precipitated proteins were washed three times with lysis buffer followed by one wash with the kinase buffer (50 mM HEPES pH 7.5, 10 mM MgCl₂ and 1 mM DTT) and subsequently resuspended in 20 µl kinase buffer containing 5 µCi [γ-³²P]ATP (PerkinElmer, Santa Clara, USA), 1 µg histone H1 (New England Biolabs, Ipswich, USA) and the respective concentration of inhibitor. After incubation for 60 minutes at 30 °C, the supernatant was boiled in sample buffer containg 60 mM Tris/HCl pH6.8, 10% Glycerol, 2% SDS, 5% β-mercaptoethanol, 0.02% Bromophenol Blue. The proteins were separated by SDS-PAGE and blotted onto nitrocellulose membrane. Thereafter, the membrane was stained with Ponceau S to check equal loading and analyzed by autoradiography.

### Kinase inhibition assays using recombinant substrates

CDK1/cyclin B and CDK2/cyclin E kinases were produced in Sf9 insect cells via baculoviral infection, while CDK5/p35, CDK7/cyclin H/MAT1, and CDK9/cyclin T1 were purchased from ProQinase (Freiburg, Germany) and assayed as described previously (35). The kinase reactions were assayed with 1 mg/ml histone H1 (for CDK2 and CDK5) or (YSPTSPS)₂KK peptide (for CDK7 and CDK9) in the presence of 15/0.15/1.5/1.5 mM ATP (for CDK2/CDK5/CDK7CDK9), 0.05 mCi [γ-³³P]ATP and of the test compound in a final volume of 10 ml. The reaction buffer contained 60 mM HEPES-NaOH, pH 7.5, 3 mM MgCl₂, 3 mM MnCl₂, 3 mM Na-orthovanadate, 1.2 mM DTT, 2.5 mg/50 ml PEG20.000. The reactions were stopped by adding 5 ml of 3% H₃PO₄. Aliquots were spotted onto P-81 phosphocellulose (Whatman, GE Healthcare Biosciences, Pittsburgh, USA), washed 3 times with 0.5% H₃PO₄ and finally air-dried. Kinase inhibition was quantified using a FLA-7000 digital image analyzer (Fujifilm, Tokyo, Japan). The concentration of the test compounds required to decrease the CDK activity by 50% was determined from dose-response curves and designated as IC₅₀.

### Analysis of proliferation

6 x 10⁴ cells were seeded on 12-well plates and incubated with inhibitors at different concentrations for 3 days. Cell numbers were determined at various time points after trypsinization using a cell counter (CASY, Schärfe Systems, Reutlingen, Germany). Three independent experiments were performed in triplicates.

### Clonogenic survival assay

To analyze the clonogenic growth behavior of cell populations on tissue culture plastic, 500 cells were seeded in a 6-well plate and, either untreated or pretreated with BA-12 or BP-14 for 24 hours, incubated with standard medium for 10 days at 37 °C and 5% CO₂. Colonies were fixed with methanol/acetic acid (3:1) and stained with 0.25% crystal violet. The crystal violet of fixed cells was solubilized with 1% SDS and the absorbance was photometrically determined at 560 nm.

### Cell Proliferation analyzed by 5-Bromo-2'-deoxy-uridine incorporation

BrdU incorporation into cell nuclei directly indicates cell proliferation. Cultured cells were grown in medium containing 10 µM 5-bromo-2'-deoxy-uridine (BrdU) for 1 hour. After removing labeling medium, cells were fixed and DNA denatured with a fixing/denaturing solution containing 2 M HCl for 30 minutes at 37 °C. To analyze BrdU incorporation *in vivo,* 200 µl Ringer solution containing 1 mg BrdU was intraperitoneally injected into xenografted mice 2 hours prior to analysis. Mice were sacrificed and tumor tissue was fixed in 4% formaldehyde and processed for immunohistochemistry. The incorporation of BrdU into cellular DNA was detected using a monoclonal anti-BrdU antibody (Sigma, St.Louis, USA) followed by incubation with a peroxidase-conjugated secondary antibodies (Calbiochem, LaJolla, USA) at dilutions of 1:10,000.

### Flow cytometry

The analysis of cellular DNA content was performed with a multicolor BD LSRFortessa cell analyzer (Becton Dickinson, Franklin Lakes, USA). Prior to the cytofluorometric measurement, about 5 x 105 cells were washed with phosphate buffered saline (PBS), fixed in 70% ethanol, washed again with PBS and treated with 100 µg RNAse A/50 µg propidium iodide per ml for 10 minutes to stain cellular DNA. The percentage of cells in the various cell cycle positions were calculated using a software package from the same manufacturer.

### Determination of long-term chemosensitivity

Hepatoma cells were continuously cultivated in the presence of BA-12 or BP-14 at concentrations lower that than the IC₅₀ (1/2 IC₅₀, 1/4 IC₅₀, 1/8 IC₅₀ and 1/16 IC₅₀). The selection of chemoresistant cells was monitored every 6 weeks by the determination of IC₅₀ values using the MTT assay. HCC cells showing higher IC₅₀ values after treatment with inhibitors as compared to untreated cells are considered as chemoresistant.

### Immunoblotting

Immunoblotting was performed as described previously (36). The primary antibodies were used at the dilutions: anti-phospho-Ser5 RNA Pol II (CDK7; Bethyl Laboratories, Montgomery, USA), 1:1,000; anti-phospho-Ser2 RNA Pol II (CDK9; Bethyl Laboratories, Montgomery, USA), 1:1,000; anti-RNA Pol II (Santa Cruz Biotechnology, Santa Cruz, USA), 1:1,000; anti-PARP (Cell Signaling Technology, Beverly, USA), 1:1,000; anti-β-actin (Sigma, St.Louis, USA), 1:2.500. Horseradish peroxidase-conjugated secondary antibodies (Calbiochem, LaJolla, USA) were used at dilutions of 1:10,000.

### Xenografted tumor formation and drug intervention

5x10⁶ human hepatoma cells were re-suspended in 100 µl Ringer solution and subcutaneously injected into severe combined immunodeficient (SCID) mice (Harlan Laboratories, San Pietro, Italy). Tumor volume was determined as described (36). Pharmacological intervention was performed in tumor-bearing mice for 17 days by daily intraperitoneal injection of either 5 mg/kg BA-12 or 1 mg/kg BP-14 in 100 µl of 0.01% DMSO. Control tumor-bearing mice were daily intraperitoneally injected with 100 µl of 0.01% DMSO only. All animal experiments were performed according to the Austrian guidelines for animal care and protection.

### Diethylnitrosamine-induced liver cancer and drug intervention

To initiate tumor development in the liver, 14-day-old C57BL/6J mice were intraperitoneally injected with a single dose of diethylnitrosamine (DEN, 25 mg/kg). After 8 month, pharmacological intervention was administrated in DEN-induced mice by 3 cycles of treatment with compounds for 10 days and a release from compounds for 7 days between the cycles. Either 5 mg/kg BA-12 or 1 mg/kg BP-14 was intraperitoneally injected in 100 µl of 0.01% DMSO. Control mice obtained 100 µl solvent only. Thereafter, mice were sacrificed and livers were fixed in 4% formaldehyde. Two researchers independently scored the diameters of neoplasia that could be monitored at the liver surface. Cancerous nodules with a diameter of up to 1 cm, covering more than 97% of all visible hepatomas, were included into the analysis. Less than 3% of neoplasia with bigger diameters was excluded due to the assumption that large tumors do not properly respond to drug treatment. All animal experiments were performed according to the Austrian guidelines for animal care and protection.

### Immunohistochemistry

Mice were sacrificed and tumors were fixed as described (37). 4 µm thick, paraffin-embedded sections were stained with hematoxylin and eosin (H&E). For immunohistochemistry, sections were stained with anti-BrdU (Sigma, St. Louis, USA), 1:200. Biotinylated secondary antibodies were used at 1:200. The immunoperoxidase procedure was performed using a Vectastain Elite ABC kit (Vector Laboratories, CA, USA) as described by the manufacturer.

### Statistical analysis

Data were expressed as means ± standard deviation (SD). The statistical significance of differences was evaluated using an unpaired, non-parametric Student's t-test. Significant differences between experimental groups were * p<0.05, ** p<0.01 or *** p<0.005.

### Results

### Cytotoxicity and kinase specificity of BP-14

Cell viability assays showed strong cytotoxic effects of BP-14 on human HepG2 and PLC hepatoma cells as well as on the established HCC cell lines Hep3B and 3sp (Fig. 7A). Evaluation of dose-response curves revealed IC₅₀ values below 0.5 µM in the various HCC cell lines (Table 11). Kinase assays using cell-free extracts showed that BP-14 significantly reduced CDK1/CDK2 activity at concentrations of 0.03 µM (Fig. 7B). In addition, treatment of HepG2 and PLC cells with BP-14 below 1 µM resulted in a strong reduction of RNA polymerase II phosphorylation on serine 5 (CDK7) and serine 2 (CDK9), suggesting inhibition of CDK7/CDK9 activity (Fig. 7C). Quantification of CDK inhibition using recombinant CDK substrates displayed IC₅₀ values of BP-14 between 0.01 to 0.05 µM including antagonizing effects on CDK5 (Table 12), thus corroborated the data obtained by cell-free extracts. Together, these results suggest that BP-14 is highly potent cytotoxic compounds on HCC cell lines by the specific inhibition of CDK1/CDK2/CDK5/CDK7 and CDK9.

**TABLE 11. Evaluation of dose-response curves (IC₅₀) in the various HCC cell lines**

| **Cell line** | **IC₅₀ (µM)** |
|---|---|
| | **BP14** |
| HepG2 | 0.12 |
| PLC | 0.02 |
| Hep3B | 0.08 |
| 3sp | 0.48 |

**Table 12. Quantification of CDK inhibition using recombinant CDK substrates - IC₅₀ values (µM).**

| **Protein kinase** | **IC₅₀ (µM)** |
|---|---|
| | **BP14** |
| CDK1/cyklin B | 0.050 |
| CDK2/cyklin E | 0.010 |
| CDK5/p25NCK | 0.015 |
| CDK9/cyklin T | 0.007 |

### BP-14 abrogates clonogenicity and represses cell cycle progression

We observed a more than 15-fold reduction of clonogenic growth behavior after treatment of HepG2 and PLC cells with 0.2 µM BP-14 (Fig. 8A). Analysis of DNA synthesis revealed that treatment of HepG2 or PLC cells with 1 µM of BP-14 decreased BrdU incorporation more than 2-fold as compared to control (Fig. 8B). Proliferation kinetics showed a cytostatic effect of BP-14 at 0.2 µM in both HepG2 and PLC cells as well as in Hep3B hepatoma cells (Fig. 9). Accordingly, BP-14 was able to induce the accumulation of HepG2 and PLC cells in the G2 phase of the cell cycle (Fig. 8C). These data suggest that BP-14 acts anti-proliferative by blocking DNA replication and by arresting HCC cells in the G2 phase of the cell cycle.

### BP-14 induces apoptosis in hepatoma cells rather than in primary human hepatocytes (PHHs)

We examined apoptosis induced by BP-14 in HepG2 cells that harbor wild type p53 and in PLC cells expressing full length but mutated p53 (Ferlay et al. Int J Cancer. 2010;127(12):2893-917). Administration of 0.2 µM BP-14 induced cleavage of PARP and p53 expression in HepG2 cells and in p53-mutated PLC cells (Fig. 10A). Yet, BP-14 failed to induce PARP processing in PHHs which are the cellular origin of hepatoma (Fig. 10B). Accordingly, BP-14 exhibited an IC₅₀ value of 20.08 µM in PHHs, which was more than 90-fold higher as compared to HepG2 cells (Tab. 11). These data show that BP-14 induces apoptosis of HCC cells at low concentration in a p53-independent fashion and fails to execute cytotoxic effects in PHHs.

### Long-term cytotoxicity of BP-14 in HCC cells

We analyzed whether BP-14 displays changes in cytotoxic effects by treating hepatoma cells at the half of their IC₅₀ concentrations as well as at serial dilutions of the IC₅₀ for up to 9 month. If a decrease in chemosensitivity occurs by the gain of resistance mechanisms, HCC cells must augment IC₅₀ values. Most notably, we observed that IC₅₀ values were maintained in hepatoma cells with very minor alterations during sustained drug exposure (Tab. 13). These data show that the cytotoxic effects of BP-14 on HCC cells are maintained upon persistent drug treatment, suggesting that hepatoma cells fail to acquire chemoresistance by BP-14.

**Table 13. Sustained cytotoxicity in HCC cell lines after long-term exposure to BP-14.**

| | **IC₅₀ for BP14 (µM)** | |
|---|---|---|
| | **HepG2** | **Hep3B** |
| **control (µM)** | 0.32 | 0.53 |
| **1/2 IC₅₀ (µM)** | 0.59 | 0.80 |
| **1/4 IC₅₀ (µM)** | 0.45 | 0.45 |
| **1/8 IC₅₀ (µM)** | 0.39 | 0.38 |
| **1/16 IC₅₀ (µM)** | 0.27 | 0.42 |

### Inhibition of xenograft models and DEN-induced hepatoma by BP-14

We assessed hepatoma xenograft models derived from HepG2 and PLC cells. Tumor-bearing mice were injected with BP-14 at the maximum tolerated dose (MTD; 1 mg/kg). Administration of BP-14 resulted in strongly reduced tumor volumes of xenografts generated by HepG2 and PLC cells (Fig. 11A). BP-14 even led to regression of PLC tumors. Evaluation of S-phase-positive cells in HepG2- and PLC-derived tumors by BrdU incorporation into DNA revealed a 2-fold decrease after exposure to BP-14 (Fig. 11B and 11C).

We further analyzed the ability of BP-14 to inhibit endogenous liver cancer formation that was chemically induced by the hepatotoxin DEN. Treatment modalities of DEN-induced mice included 3 cycles of treatment at MTD of BP-14 for 10 days with interim breaks of 7 days (Fig. 12A). Evaluation of tumor nodules that are observed on the surface of cancerous livers revealed that BA-12 causes a 1.4-fold decrease of tumor nodules as compared to untreated control mice. Intervention with BP-14 showed comparable anti-cancer effects by a 1.3-fold decline of DEN-induced hepatoma (Fig. 12B and 12C). In summary, these data suggest that both BA-12 and BP-14 exhibit strong anti-hepatoma activities *in vivo* as observed in xenograft models as well as in endogenous liver cancer.

### EXAMPLE 26 Dry Capsules

5000 capsules, each of which contains 0.25 g of a compound of the formula I as an active ingredient, are prepared as follows:

**Composition**

| | |
|---|---|
| Active ingredient | 1250 g |
| Talc | 180 g |
| Wheat starch | 120 g |
| Magnesium stearate | 80 g |
| Lactose | 20 g |

Preparation process: The powdered substances mentioned are pressed through a sieve of mesh width 0.6 mm. Portions of 0.33 g of the mixture are transferred to gelatine capsules with the aid of a capsule-filling machine.

### EXAMPLE 27 Soft Capsules

5000 soft gelatine capsules, each of which contains 0.05 g of a compound of the formula I as an active ingredient, are prepared as follows:

**Composition**

| | |
|---|---|
| Active ingredient | 250 g |
| Lauroglycol | 2 litres |

Preparation process: The powdered active ingredient is suspended in Lauroglykol^{®} (propylene glycol laurate, Gattefossé S.A., Saint Priest, France) and ground in a wet-pulveriser to a particle size of about 1 to 3 µm. Portions of in each case 0.419 g of the mixture are then transferred to soft gelatine capsules by means of a capsule-filling machine.

### EXAMPLE 28 Soft Capsules

5000 soft gelatine capsules, each of which contains 0.05 g of a compound of the formula I as an active ingredient, are prepared as follows:

**Composition**

| | |
|---|---|
| Active ingredient | 250 g |
| PEG 400 | 1 litre |
| Tween 80 | 1 litre |

Preparation process: The powdered active ingredient is suspended in PEG 400 (polyethylene glycol of Mr between 380 and about 420, Sigma, Fluka, Aldrich, USA) and Tween^{®} 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 mm. Portions of in each case 0.43 g of the mixture are then transferred to soft gelatine capsules by means of a capsule-filling machine.

## Claims

1. 2-Substituted-6-biarylmethylamino-9-cyclopentyl-9*H*-purines of the general formula **I** 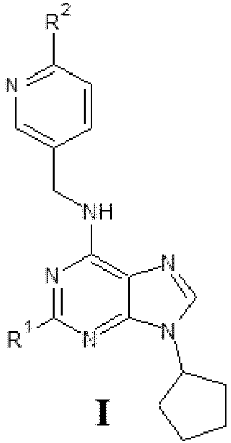 wherein
R1 is selected from the group consisting of:
(4-aminocyclohexyl)amino,
(2-aminocyclohexyl)amino,
(3-aminocyclohexyl)amino,
(4-hydroxycyclohexyl)amino,
[(2R)-1-hydroxybutan-2-yl]amino,
(2-hydroxy-2-methylpropyl)amino,
(3-hydroxy-3-methylbutyl)amino,
[(3S)-2-hydroxy-2,4-dimethylpent-3-yl]amino,
[(1S)-1-(dimethylamino)-2-hydroxyethyl] amino,
[(3R)-2-hydroxypent-3-yl]amino,
(3-hydroxypropyl)amino,
(2-aminoethyl)amino,
(3-aminopropyl)amino,
(2-aminopropyl)amino,
and
R2 is selected from the group consisting of
substituted phenyl, wherein the substituents are in any positions and are independently selected from the group consisting of OH, OCH₃, NH₂, Cl, Br, F, I, COOH, NO₂,
2-pyridyl,
3-pyridyl,
4-pyridyl,
2-furanyl
3-furanyl,
thien-2-yl,
thien-3-yl,
pyrazol-1-yl,
pyrazol-3-yl,
pyrazol-4-yl,
pyrrol-1-yl,
and the pharmaceutically acceptable salts thereof, in particular salts with alkali metals, ammonium or amines, or addition salts with acids.

2. 2-Substituted-6-biarylmethylamino-9-cyclopentyl-9*H*-purines according to claim 1 of the general formula I in the form of (R) or (S) isomers in case of chirality in position R2.

3. 2-Substituted-6-biarylmethylamino-9-cyclopentyl-9*H*-purine derivatives according to claim 1 or 2 for use as medicaments.

4. 2-Substituted-6-biarylmethylamino-9-cyclopentyl-9*H*-purine derivatives according to claim 1 or 2 for use in inhibiting angiogenesis in mammalian cells.

5. 2-Substituted-6-biarylmethylamino-9-cyclopentyl-9*H*-purine derivatives according to claim 1 or 2 for use in suppression of inflammation.

6. 2-Substituted-6-biarylmethylamino-9-cyclopentyl-9*H*-purine derivatives according to claim 1 or 2 for use in the treatment of cancer disorders.

7. 2-Substituted-6-biarylmethylamino-9-cyclopentyl-9*H*-purine derivatives according to claim 1 or 2 for use in the treatment of cancer disorders selected from the group comprising hepatocellular carcinoma and metastatic hepatocellular carcinoma.

8. A pharmaceutical composition, **characterized in that** it contains at least one 2-substituted-6-biarylmethylamino-9-cyclopentyl-9*H*-purine derivative according to claim 1 or 2 and a pharmaceutically acceptable carrier.

9. The pharmaceutical composition according to claim 8, **characterized in that** it further contains at least one further anti-tumor agent, preferably cis-platinum, doxorubicin or sorafenib.

## Patentansprüche

1. 2-Substituierte-6-Biarylmethylamino-9-cyclopentyl-9H-purine der allgemeinen Formel I 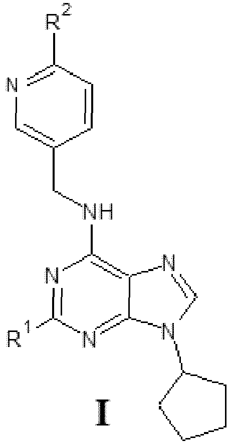 worin
R1 ausgewählt ist aus der Gruppe bestehend aus:
(4-Aminocyclohexyl) amino,
(2-Aminocyclohexyl) amino,
(3-Aminocyclohexyl) amino,
(4-Hydroxycyclohexyl) amino,
[(2R) -1-Hydroxybutan-2-yl] amino,
(2-Hydroxy-2-methylpropyl) amino,
(3-Hydroxy-3-methylbutyl) amino,
[(3S)-2-Hydroxy-2,4-dimethylpent-3-yl] amino,
[(1S)-1-(Dimethylamino)-2-hydroxyethyl] amino,
[(3R)-2-Hydroxypent-3-yl] amino,
(3-Hydroxypropyl) amino,
(2-Aminoethyl) amino,
(3-Aminopropyl) amino,
(2-Aminopropyl) amino,
und
R2 ausgewählt ist aus der Gruppe bestehend aus
Substituiertes Phenyl, wobei die Substituenten in beliebigen Positionen vorliegen und unabhängig voneinander ausgewählt sind aus der Gruppe bestehend aus OH, OCH₃, NH₂, Cl, Br, F, I, COOH, NO₂,
2-Pyridyl,
3-Pyridyl,
4-Pyridyl,
2-Furanyl
3-Furanyl,
Thien-2-yl,
Thien-3-yl,
Pyrazol-1-yl,
Pyrazol-3-yl,
Pyrazol-4-yl,
Pyrrol-1-yl,
und deren pharmazeutisch annehmbare Salze, insbesondere Salze mit Alkalimetallen, Ammonium oder Aminen, oder Additionssalze mit Säuren.

2. 2-Substituierte-6-Biarylmethylamino-9-cyclopentyl-9H-purine gemäß Anspruch 1 der allgemeinen Formel I in Form von (R) oder (S)-Isomeren im Falle der Chiralität in Position R2.

3. 2-Substituierte-6-Biarylmethylamino-9-cyclopentyl-9H-purin-Derivate gemäß Anspruch 1 oder 2 zur Verwendung als Arzneimittel.

4. 2-Substituierte-6-Biarylmethylamino-9-cyclopentyl-9H-purin-Derivate nach Anspruch 1 oder 2 zur Verwendung bei der Hemmung der Angiogenese in Säugetierzellen.

5. 2-Substituierte-6-Biarylmethylamino-9-cyclopentyl-9H-purin-Derivate nach Anspruch 1 oder 2 zur Verwendung bei der Unterdrückung von Entzündungen.

6. 2-Substituierte-6-Biarylmethylamino-9-cyclopentyl-9H-purin-Derivate gemäß Anspruch 1 oder 2 zur Verwendung bei der Behandlung von Krebserkrankungen.

7. 2-Substituierte-6-Biarylmethylamino-9-cyclopentyl-9H-purin-Derivate nach Anspruch 1 oder 2 zur Verwendung bei der Behandlung von Krebserkrankungen, ausgewählt aus der Gruppe umfassend hepatozelluläres Karzinom und metastasiertes hepatozelluläres Karzinom.

8. Pharmazeutische Zusammensetzung, **dadurch gekennzeichnet, daß** sie mindestens ein 2-substituiertes-6-Biarylmethylamino-9-cyclopentyl-9H-purin-Derivat nach Anspruch 1 oder 2 und einen pharmazeutisch verträglichen Träger enthält.

9. Pharmazeutische Zusammensetzung nach Anspruch 8, **dadurch gekennzeichnet, dass** sie weiterhin mindestens ein weiteres Antitumormittel, vorzugsweise cis-Platin, Doxorubicin oder Sorafenib enthält.

## Revendications

1. 2-Substitués 6-biarylméthylamino-9-cyclopentyl-9H-purines de formule générale I 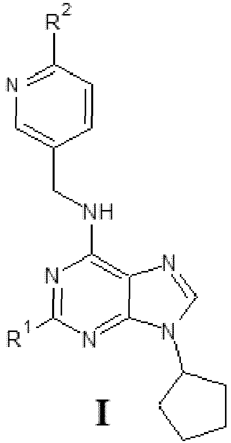 où
R1 est choisi dans le groupe consistant en:
(4-aminocyclohexyl) amino,
(2-aminocyclohexyl) amino,
(3-aminocyclohexyl) amino,
(4-hydroxycyclohexyl) amino,
[(2R)-1-hydroxybutan-2-yl] amino,
(2-hydroxy-2-méthylpropyl) amino,
(3-hydroxy-3-méthylbutyl) amino,
[(3S)-2-hydroxy-2,4-diméthylpent-3-yl] amino,
[(1S)-1-(diméthylamino)-2-hydroxyéthyl] amino,
[(3R)-2-hydroxypent-3-yl] amino,
(3-hydroxypropyl) amino,
(2-aminoéthyl) amino,
(3-aminopropyl) amino,
(2-aminopropyl) amino,
et
R2 est choisi dans le groupe consistant en
phényle substitué, où les substituants sont dans n'importe quelle position et sont choisis indépendamment dans le groupe consistant en OH, OCH₃, NH₂, Cl, Br, F, I, COOH, NO₂,
2-pyridyle,
3-pyridyle,
4-pyridyle,
2-furanyle,
3-furanyle,
thién-2-yle,
thién-3-yle,
pyrazol-1-yle,
pyrazol-3-yle,
pyrazol-4-yle,
pyrrol-1-yle,
et leurs sels pharmaceutiquement acceptables, en particulier des sels avec des métaux alcalins, de l'ammonium ou des amines, ou des sels d'addition avec des acides.

2. 2-Substitués 6-biarylméthylamino-9-cyclopentyl-9H-purines de formule générale I selon la revendication 1 sous la forme de (R) ou (S) dans le cas des isomères de chiralité en position R2.

3. Dérivés de 6-biarylméthylamino-9-cyclopentyl-9H-purine 2-substitués selon la revendication 1 ou 2 pour une utilisation en tant que médicaments.

4. Dérivés de 6-biarylméthylamino-9-cyclopentyl-9H-purine 2-substitués selon la revendication 1 ou 2 pour une utilisation dans l'inhibition de l'angiogenèse dans des cellules de mammifères.

5. Dérivés de 6-biarylméthylamino-9-cyclopentyl-9H-purine 2-substitués selon la revendication 1 ou 2 pour une utilisation dans la suppression de l'inflammation.

6. Dérivés de 6-biarylméthylamino-9-cyclopentyl-9H-purine 2-substitués selon la revendication 1 ou 2 pour une utilisation dans le traitement de maladies cancéreux.

7. Dérivés de 6-biarylméthylamino-9-cyclopentyl-9H-purine 2-substitués selon la revendication 1 ou 2 pour une utilisation dans le traitement de maladies cancéreux choisis dans le groupe comprenant le carcinome hépatocellulaire et le carcinome hépatocellulaire métastatique.

8. Composition pharmaceutique, **caractérisée en ce qu'**elle contient au moins un dérivé 6-biarylméthylamino-9-cyclopentyl-9H-purine 2-substitué selon la revendication 1 ou 2 et une substance auxiliaire support pharmaceutiquement acceptable.

9. Composition pharmaceutique selon la revendication 8, **caractérisée en ce qu'**elle contient en outre au moins un autre agent antitumoral, de préférence cis-platine, doxorubicine ou sorafénib.
